(19)

Europäisches
Patentamt
European
Patent Office
Office européen
des brevets

(11) **EP 4 435 794 A2**

(12) **EUROPEAN PATENT APPLICATION**

(43) Date of publication:
**25.09.2024 Bulletin 2024/39**

(21) Application number: **24170209.1**

(22) Date of filing: **02.11.2021**

(51) International Patent Classification (IPC):
***G16C 20/70*** *(2019.01)*

(52) Cooperative Patent Classification (CPC):
**G16B 15/00; G16B 15/30; G16B 40/20;** Y02A 90/10

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.11.2020 US 202063108716 P**

(62) Document number(s) of the earlier application(s) in
accordance with Art. 76 EPC:
**21823693.3 / 4 238 095**

(27) Previously filed application:
**02.11.2021 US PCT/US2021/057731**

(71) Applicant: **Regeneron Pharmaceuticals, Inc.
Tarrytown, NY 10591 (US)**

(72) Inventors:
• **ARORA, Jayant
Edison, New Jersey 08820 (US)**

• **TANG, Xiaolin
Tappan, New Jersey 07675 (US)**
• **SHAMEEM, Mohammed
Nanuet, New York 10954 (US)**
• **TAFAZZOL, Alireza
San Francisco, CA 94109 (US)**

(74) Representative: **HGF
HGF Limited
1 City Walk
Leeds LS11 9DX (GB)**

Remarks:
•This application was filed on 15-04-2024 as a
divisional application to the application mentioned
under INID code 62.
•Claims filed after the date of filing of the application
/ after the date of receipt of the divisional application
(Rule 68(4) EPC).

(54) **METHODS AND SYSTEMS FOR BIOTHERAPEUTIC DEVELOPMENT**

(57)    Disclosed are methods comprising determining experimental data associated with one or more monoclonal antibodies (mAbs), determining computationally-derived data associated with the one or more mAbs, wherein the computationally-derived data comprises one or more computational parameters weighted based on accessible surfaces (ASAs) of one or more residues of the one or more mAbs, determining, based on the experimental data and the computationally-derived data, a plurality of candidate predictive models, determining an optimal predictive model from the plurality of candidate predictive models, and outputting the optimal predictive model.

**FIG. 1**

**Description**

**CROSS REFERENCE TO RELATED PATENT APPLICATION**

This application claims priority to U.S. Provisional Application No. 63/108,716 filed November 2, 2020, herein incorporated by reference in its entirety.

**BACKGROUND**

Ever since the first FDA-approved monoclonal antibody (mAb) Muromonab, a murine CD3 specific IgG2a monoclonal antibody indicated for acute organ transplant rejection, in 1986, more than 64 mAbs have been approved by FDA. The popularity of this therapeutic platform is also evidenced by the ascending number of ongoing clinical trials each year with their use expanding into a broad spectrum of different therapeutic portfolios. Therapeutic mAbs are most commonly administered through three routes of administration, intravenous (IV), intramuscular (IM) and subcutaneous (SC) injections; this choice is based on various contributing factors including their safety, efficacy, patient satisfaction, and pharmacoeconomics. IV administration can be delivered at a controllable high dose usually at a clinic and is thus usually costlier for patients as well as clinicians. A shift from the IV route to SC for most immunoglobulins has the potential to reduce overall health care costs as it enables at-home self-administration by the patients or faster in-clinic administration by heath care professionals, relieves economic burden off the health care system by reducing longer in-patient visits and improves overall quality of healthcare. Despite offering many advantages over IV administration, SC route presents some significant challenges to drug product development and drug administration. The primary disadvantage of SC administration is the inherent resistance and volume restriction of the extracellular matrix, which requires highly concentrated antibody solutions ($\geq$150 mg/mL) to be administered in a limited injection volume (~2-3 mL) for optimal PK/PD outcome and user convenience.

A highly concentrated antibody solution is challenging to develop in a drug product as high protein concentrations can result in significant technical challenges such as high solution viscosity and protein aggregation rates. Highly viscous antibodies also lead to difficulties related to manufacturing processes and drug delivery. Protein aggregation may lead to a reduction in antibody activity and can impact protein pharmacokinetics and safety because of their greater immunogenicity potential. Therefore, appropriate formulations should be developed for highly concentrated therapeutic antibodies to stabilize their structures both colloidally and conformationally, thereby, decreasing viscosity and aggregation propensity to ensure both acceptable shelf life and compatibility with the manufacturing process.

The traditional approaches to develop high concentration protein formulations generally involve empirical methods utilizing an extensive array of biophysical and analytical techniques. For example, solution viscosity can be measured by direct viscosity measurements and experimentally predicted by established tools such as osmotic second virial coefficient ($B_{22}$) measurement, and diffusion interaction parameter ($K_D$) measurements. Such tools for example, $B_{22}$ measurements require a significant amount of material and are fairly labor intensive. Protein aggregation and association are considered to be an outcome of the interplay between conformational stability (i.e., macro and micro perturbations in protein structure) and colloidal stability (i.e., native intermolecular interactions).

Various established biophysical and analytical tools and approaches are regularly used to assess overall protein stability and predict individual contributions of conformational and colloidal stability. These stabilities can be measured and predicted by various established techniques that enable quantification of thermal, agitation, and freeze-thaw stresses. Stability prediction approaches such as thermal stress stability studies, measurement of thermal denaturation temperature ($T_m$), chemical denaturation temperature, aggregation temperature ($T_{agg}$), cloud-point temperature ($T_{cloud}$), direct surface hydrophobicity measurement using hydrophobic interaction chromatography, zeta potential, and high order structure estimation. All of these techniques require physical material and some techniques are cumbersome and time-consuming.

Moreover, experimentally developed prediction models fail most of the time. Most of the above-mentioned techniques to measure and predict viscosity and aggregation rates are costly, time consuming, and require physical material. Hence, development of novel experimental and/or *in silico* tools to predict viscosity values and aggregation propensity or to rank antibodies based on their developability is indispensable for rapid screening of many mAb candidates during early formulation development and discovery.

Physical properties obtainable from antibody sequences, homology models, and molecular dynamics (MD) simulations of individual antibody molecules can be used as parameters to develop predictive models or rank ordering schemes. These models can be used to predict viscosity, conformational stability, colloidal stability, and manufacturability. These rapid material-free tools can also enable more molecular insight into antibody molecules and their interactions.

Sharma et al. developed a predictive model for viscosity based on the variable domain Fv of fourteen IgG1 homology models. Physical parameters including, but not limited to, charge and hydrophobicity were correlated to the measured viscosity values in a principal component regression model. Agrawal et al. also developed a viscosity scoring function ranking IgG1 antibodies based on the partial charge of the surface-exposed residues of the Fv region in the homology models and suggested thresholds to differentiate highly viscous antibodies from the rest.

Tomar et al. predicted concentration-dependent viscosity curves of the antibody solutions based on electrostatic and hydrophobic descriptors obtained from full-length homology models of sixteen IgG1, IgG2, and IgG4 antibodies. The hydrophobic surface area of full-length antibody and charges on Fv and hinge regions were used to predict the slope of the linearized concentration-dependent viscosity curve.

Moreover, various methods have been developed by researchers in the field to predict aggregation prone regions of peptides and therapeutic proteins. TANGO statistical mechanics algorithm was developed based on the physico-chemical principles of β-sheet formation to predict sequence-based aggregation. A web-based tool, Waltz, was designed to use a position-specific scoring matrix to identify amyloid-forming regions in a protein sequence. Chennamsetty et al. developed a method based on dynamic exposure of hydrophobic patches obtained from antibody atomistic simulations to predict aggregation prone regions. A comprehensive list of computational methods developed to predict therapeutic protein aggregation and aggregation-prone regions can be found in the published book chapters and review papers.

Overall, there is a need for more robust and predictive models for viscosity and aggregation propensity to facilitate drug product development.

## BRIEF SUMMARY

Described are methods comprising determining experimental data associated with one or more monoclonal antibodies (mAbs), determining computationally-derived data associated with the one or more mAbs, wherein the computationally-derived data comprises one or more computational parameters weighted based on accessible surfaces (ASAs) of one or more residues of the one or more mAbs, determining, based on the experimental data and the computationally-derived data, a plurality of candidate predictive models, determining an optimal predictive model from the plurality of candidate predictive models, and outputting the optimal predictive model.

Also described are methods comprising receiving computationally-derived data associated with a monoclonal antibody (mAb), providing, to a predictive model, the computationally-derived data, and determining, based on the predictive model, a viscosity score associated with the mAb.

Also described are methods comprising receiving computationally-derived data associated with a monoclonal antibody (mAb), and providing, to a predictive model, the computationally-derived data, and determining, based on the predictive model, an aggregation score associated with the mAb.

Additional advantages of the disclosed method and compositions will be set forth in part in the description which follows, and in part will be understood from the description, or may be learned by practice of the disclosed method and compositions. The advantages of the disclosed method and compositions will be realized and attained by means of the elements and combinations particularly pointed out in the appended claims. It is to be understood that both the foregoing general description and the following detailed description are exemplary and explanatory only and are not restrictive of the invention as claimed.

## BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.

Figure 1 is a flow chart showing an example for generating a predictive model to assist in therapeutic screening and/or selection.

Figure 2 is an example block diagram for generating a predictive model.

Figure 3 is a flowchart illustrating an example training method

Figure 4 is an illustration of an exemplary process flow for using a machine learning-based classifier to determine whether a nucleotide sequence is a promoter.

Figure 5 shows an example of the diffusion interaction parameter ($K_D$) calculated based on fitting a line to the diffusion coefficients at various protein concentrations. This graph shows how $K_D$ was calculated for mAb4 as an example.

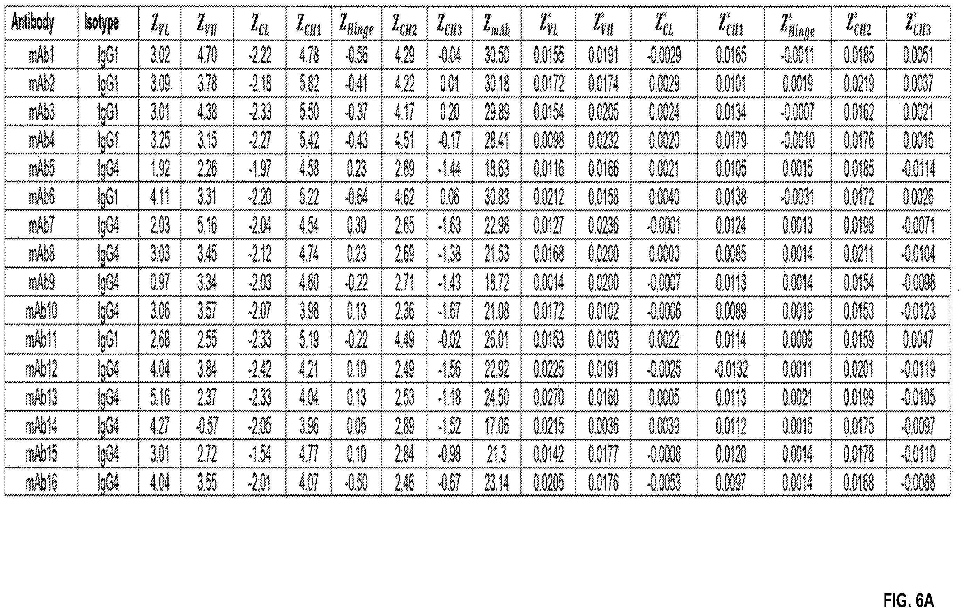

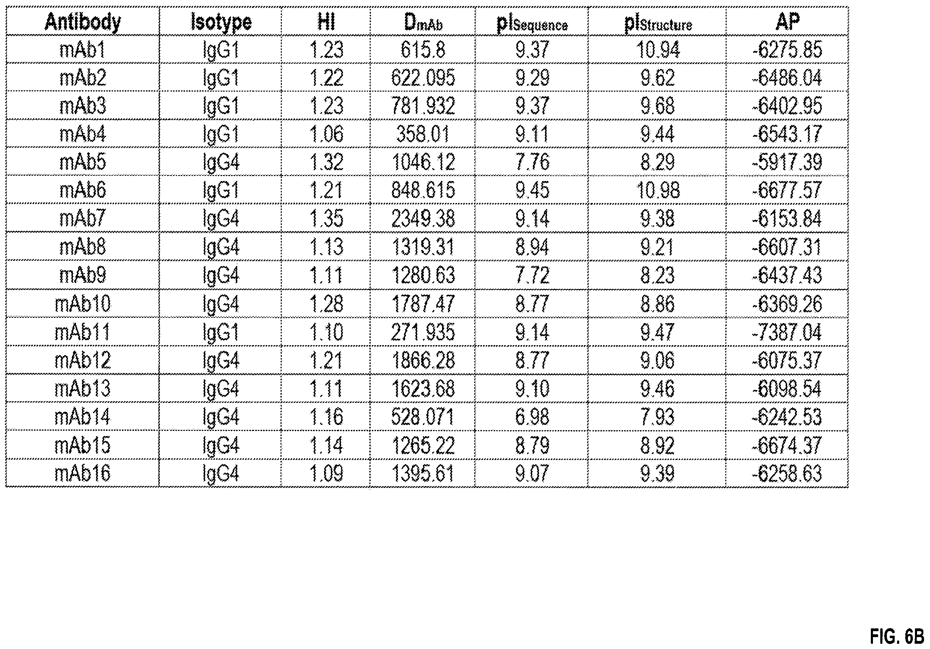

Figures 6A and 6B are tables showing computed parameters for 16 full antibody models used in this study. These physical properties were obtained from full antibody homology models to be used to develop a predictive model for protein solution viscosity. $Z_{VL}$, $Z_{VH}$, $Z_{CL}$, $Z_{CH1}$, $Z_{Hinge}$, $Z_{CH2}$, and $Z_{CH3}$ are net charges on the $V_L$, $V_H$, $C_L$, $C_H1$, hinge, $C_H2$, and $C_H3$ regions, respectively; $Z_{mAb}$ is the total antibody charge; $Z^*_{VL}$, $Z^*_{VH}$, $Z^*_{CL}$, $Z^*_{CH1}$, $Z^*_{Hinge}$, $Z^*_{CH2}$, and $Z^*_{CH3}$ are net solvent accessible surface (SAS) adjusted charges on the $V_L$, $V_H$, $C_L$, $C_H1$, hinge, $C_H2$, and $C_H3$ regions, respectively; HI is the hydrophobicity index; $D_{mAb}$ is the averaged total dipole moment of the antibody; $PI_{Sequence}$ and $PI_{Structure}$ are sequence-based and structure-based isoelectric points, respectively; and AP is the aggregation propensity predicted by Chennamsetty

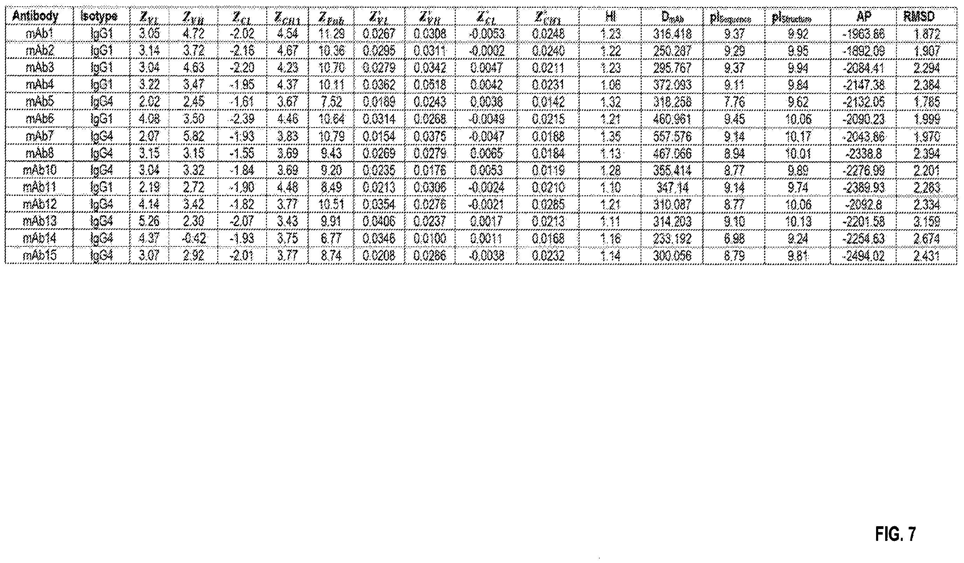

Figure 7 is a table showing computed parameters for Fab models. Computed parameters for 14 Fab models used in this study. These physical properties were obtained from Fab homology models and molecular dynamics simulations to be used to develop a predictive model for aggregation propensity. $Z_{VL}$, $Z_{VH}$, $Z_{CL}$, and $Z_{CH1}$ are net charges on the $V_L$, $V_H$, $C_L$, and $C_H1$ regions, respectively; $Z_{Fab}$ is the total Fab region charge; $Z^*_{VL}$, $Z^*_{VH}$, $Z^*_{CL}$, and $Z^*_{CH1}$ are net solvent accessible surface (SAS) adjusted charges on the $V_L$, $V_H$, $C_L$, and $C_H1$ regions, respectively; HI is the hydrophobicity index; $D_{mAb}$ is the averaged total dipole moment of the Fab region; $PI_{Sequence}$ and $PI_{Structure}$ are sequence-based and structure-based isoelectric points, respectively; AP is the aggregation propensity predicted by Chennamsetty; and RMSD is the averaged root mean square deviation (Å) obtained from Fab region molecular dynamics simulations.

Figure 8 shows the broad distribution of measured protein solution viscosity values for 16 mAbs used in this study. The IgG1 and IgG4 candidates are shown in grey and black, respectively. Based on our dataset, the IgG1 antibodies tend to show lower viscosity values when compared to IgG4 candidates.

Figures 9A, 9B, and 9C show an example of the measured values for 15 mAbs used in this study, (a) the osmotic second virial coefficient ($B_{22}$), (b) the diffusion interaction parameter ($K_D$), and (c) the strong correlation between $B_{22}$ and $K_D$ values for the current dataset as observed by high correlation coefficient (R). MAb1 was excluded from these plots because of lack of materials for the $K_D$ measurement. IgG1 and IgG4 candidates are colored in grey and black, respectively.

Figures 10A and 10B show an example of the correlation of measured values to protein solution viscosity: **(a)** the osmotic second virial coefficient ($B_{22}$), and **(b)** the diffusion interaction parameter ($K_D$). The $B_{22}$ values were measured for 16 mAbs used in this study and the $K_D$ values were measured for 15 of them as the lack of enough material for mAb1. The linear correlation coefficient (R) and the regression line are shown on each graph.

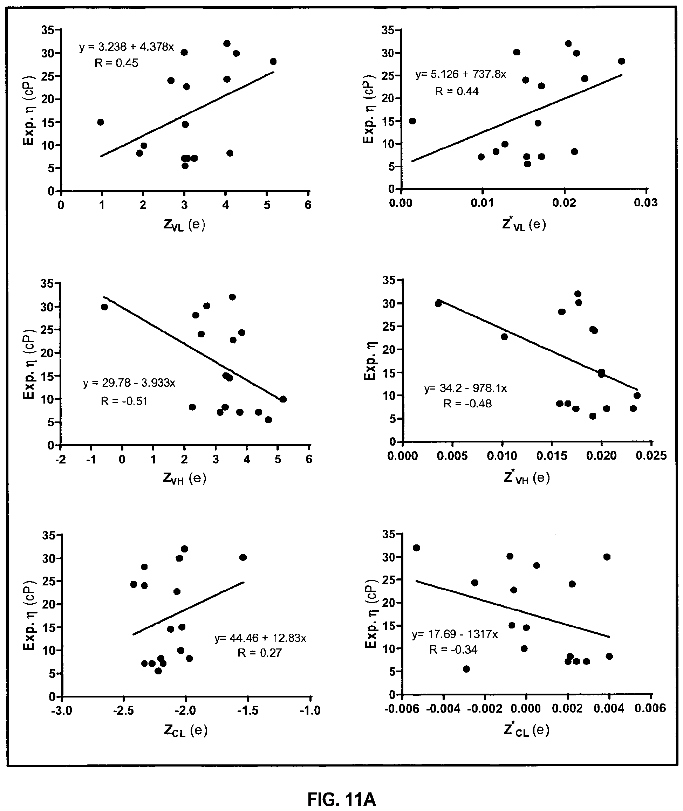

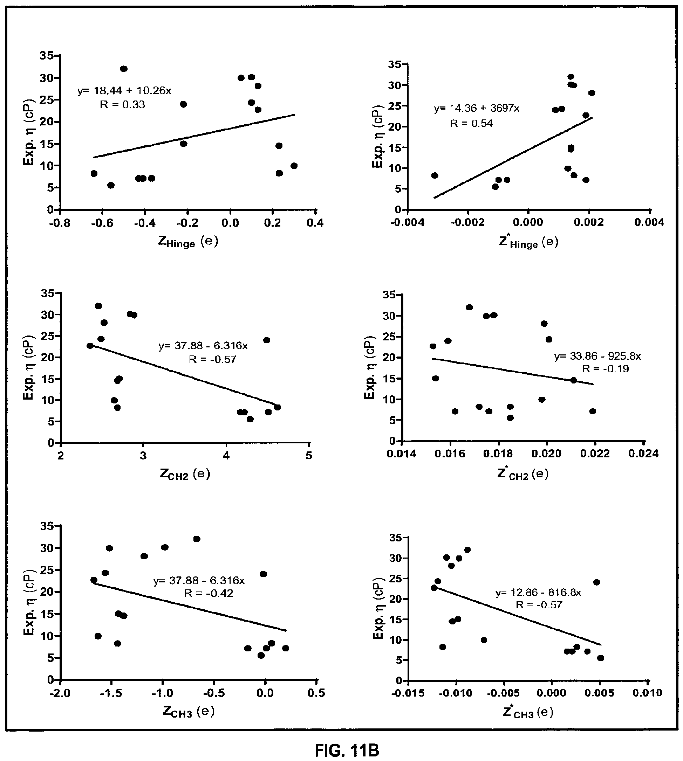

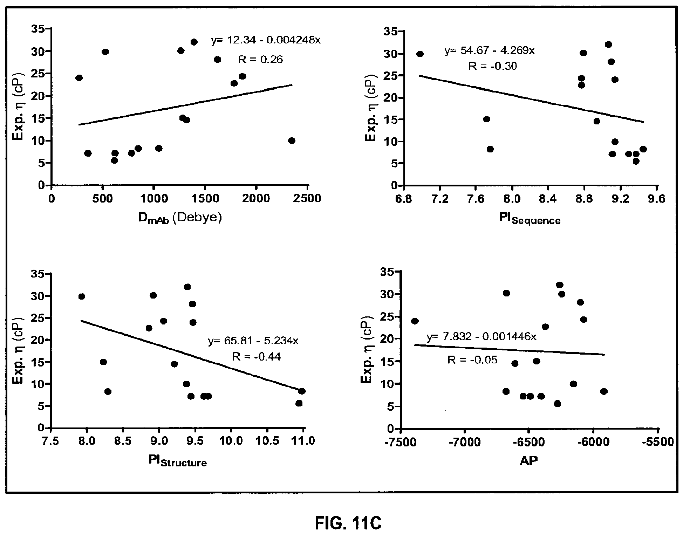

Figures 11A, 11B and 11C show the linear relationships between the experimental viscosity values and the computed parameters. The linear fitted equation and correlation coefficient (R) are shown on each plot. $Z_{VL}$, $Z_{VH}$, $Z_{CL}$, $Z_{CH1}$, $Z_{Hinge}$, $Z_{CH2}$, and $Z_{CH3}$ are net charges on the $V_L$, $V_H$, $C_L$, $C_H1$, hinge, $C_H2$, and $C_H3$ regions, respectively; $Z_{mAb}$ is the total antibody charge; $Z^*_{VL}$, $Z^*_{VH}$, $Z^*_{CL}$, $Z^*_{CH1}$, $Z^*_{Hinge}$, $Z^*_{CH2}$, and $Z^*_{CH3}$ are net solvent accessible surface (SAS) adjusted charges on the $V_L$, $V_H$, $C_L$, $C_H1$, hinge, $C_H2$, and $C_H3$ regions, respectively; HI is the hydrophobicity index; $D_{mAb}$ is the averaged total dipole moment of the antibody; $PI_{Sequence}$ and $PI_{Structure}$ are sequence-based and structure-based isoelectric points, respectively; and AP is the aggregation propensity predicted by Chennamsetty.

Figure 12 shows an example of the linear regression line between the computed predicted viscosity score (PVS) and measured viscosity values. The correlation coefficient (R) and the square of correlation coefficient ($R^2$) are shown on the graph. The area between dashed lines show the 95% confidence interval.

Figure 13 shows a representative of size-exclusion chromatography (SEC) signal for one of the antibodies used in this study, mAb3, over period of time for 0-day and 28-day incubation at 40 °C and 75% relative humidity. An increase in the high-molecular-weight (HMW) species formation is observed as a result of aggregation. The 0-day and 28-day data are shown as dotted red and solid black lines, respectively.

Figure 14 shows the relative percentage of high-molecular-weight (HMW) species formations for 7, 14, and 28 days comparing to the 0 day, %ΔHMW, for fourteen mAbs used in this study were measured by size-exclusion chromatography (SEC). The samples were incubated for 7, 14, and 28 days at 40 °C and 75% relative humidity. MAb9 and mAb16 were excluded because of limitation of materials availability.

Figure 15 shows the rate of high-molecular-weight (HMW) species formations per day, %ΔHMW/Day, as calculated based on %ΔHMW of 28-day data points divided by 28 for 14 mAbs used in this study. The IgG1 and IgG4 are colored in grey and black, respectively.

Figures 16A, 16B, and 16C show the root mean square deviations (RMSDs) of conformational structures relative to the initial structure for 14 mAbs used in this study. These conformations were obtained from molecular dynamics simulations on Fab region of each antibody for 2.0 ns as replicated three times. The first, second, and third simulations for each mAb are colored in black, red, and blue, respectively.

Figure 17 shows an example of an averaged root mean square deviation (RMSD) of backbone atoms of Fab models from the initial structures for 14 mAbs used in the current study to develop a predictive model for aggregation. The RMSD value can be used as a descriptor for the conformational stability to differentiate antibodies from each other. The RMSD values for each mAb are an average of 3 replications of molecular dynamics (MD) simulations. MAb9 and mAb16 were excluded because of limitation of materials availability.

Figures 18A, 18B, and 18C show the averaged root mean square deviations (RMSDs) over three 2.0 ns molecular dynamics simulations of Fab region in each mAb. The RMSDs in each simulation was calculated for each conformation relative to the initial structure.

Figures 19A, 19B, and 19C show a linear relationship between the measured rate of high-molecular-weight (HMW) species formation per day, %ΔHMW/Day, and the computed parameters including root mean square deviation (RMSD) obtained from molecular dynamics simulations.

Figure 20 shows an example of the linear regression line between the computed predicted aggregation score (PAS) and the measured rate of high-molecular-weight (HMW) formation per day. The correlation coefficient (R) and the square of correlation coefficient ($R^2$) are shown on the graph. The area between dashed lines show the 95% confidence interval.

Figure 21 shows correlation of predicted viscosity scores with validation experimental data.

Figure 22 shows correlation of predicted aggregation scores with validation experimental data.

Figure 23 shows an example operating environment.

Figure 24 shows an example method.

Figure 25 shows an example method.

Figure 26 shows an example method.

## DETAILED DESCRIPTION

The disclosed method and compositions may be understood more readily by reference to the following detailed description of particular embodiments and the Example included therein and to the Figures and their previous and following description.

It is understood that the disclosed method and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a," "an," and "the" include plural reference unless the context clearly dictates otherwise. Thus, for example, reference to "an antibody" includes a plurality of such antibodies, reference to "the antibody" is a reference to one or more antibodies and equivalents thereof known to those skilled in the art, and so forth.

As used herein, the term "antibody" refers to a whole antibody. An antibody is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three subdomains, CH1, CH2 and CH3. Each light chain is comprised of a light chain variable region (abbreviated herein as VL) and a light chain constant region. The light chain constant region is comprised of one subdomain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system. In some aspects, antibodies can be chimeric, monoclonal, and/or humanized.

Antibody fragments can refer to any smaller portion of a whole antibody. Antibody fragments can be described in terms of proteolytic fragments including without limitation Fv (variable region fragment), Fab (antibody binding region fragment), Fab' and F(ab')2 (antibody binding fragment plus part of the hinge region) fragments. Such fragments may be prepared by standard methods (see, e.g., Coligan et al. Current Protocols in Immunology, John Wiley & Sons, 1991-1997, incorporated herein by reference). An antibody may comprise at least three proteolytic fragments (i.e., fragments produced by cleavage with papain): two Fab fragments, each containing a light chain domain and a heavy chain domain (designated herein as a "Fab heavy chain domain") and one Fc fragment containing two Fc domains. Each light chain domain contains a VL and a CL subdomain, each Fab heavy chain domain contains a VH and a CH1 subdomain, and each Fc domain contains a CH2 and CH3 subdomain. In some aspects, antibody fragments can be chimeric, monoclonal, and/or humanized.

As used herein, the term "monoclonal antibody" or "monoclonal antibody fragment" refers to an antibody or antibody fragment obtained from a single clonal population of immunoglobulins that bind to the same epitope of an antigen. Monoclonal antibodies have the same Ig gene rearrangement and thus demonstrate identical binding specificity. Methods for preparing monoclonal antibodies are known in the art.

As used herein, "humanized monoclonal antibody" or "humanized monoclonal antibody fragment" may refer to monoclonal antibodies or fragments thereof having at least human constant regions and an antigen-binding region, such as one, two or three CDRs, from a non-human species. Humanized antibodies or fragments thereof specifically recognize antigens of interest, but will not evoke an immune response in humans against the antibody itself.

As used herein, the term "chimeric antibody" or "chimeric antibody fragment" refers to a monoclonal antibody or fragment thereof comprising a variable region from one source (e.g., species) and at least a portion of a constant region derived from a different source. In some embodiments, the chimeric antibodies comprise a murine variable region and a human constant region.

Throughout the description and claims of this specification, the word "comprise" and variations of the word, such as "comprising" and "comprises," means "including but not limited to," and is not intended to exclude, for example, other additives, components, integers or steps. In particular, in methods stated as comprising one or more steps or operations it is specifically contemplated that each step comprises what is listed (unless that step includes a limiting term such as "consisting of'), meaning that each step is not intended to exclude, for example, other additives, components, integers or steps that are not listed in the step.

During drug discovery and early development, the majority of drug candidates are initially screened and selected based on affinity and functionality. However, there are other properties and attributes that need to be considered in biotherapeutic development. For example, protein yield, viscosity, aggregation, chemical stability (e.g., susceptibility to degradation through oxidation, deamidation), formulability and immunogenicity, should form part of a comprehensive developability risk assessment. The concept of developability is used to define the suitability of a drug candidate (e.g., antibody) to be developed as a therapeutic/drug. Once the antibody has been identified and developed as a drug, it can then be administered to patients.

Disclosed are methods of predicting antibody viscosity. This viscosity prediction tool can be used early in the drug development process to enable ranking and selection of lead antibodies with reduced risk of being viscous.

Antibody drugs require highly concentrated formulations, which can result in highly viscous solutions that are challenging to handle both in manufacturing processes and in end user injections. Often times, antibody viscosity is only discovered late in drug development after there has been significant R&D investment. The methods and systems described herein can predict antibody viscosity early in the drug development process to allow for prioritizing low viscosity antibody candidates. The viscosity prediction tools are described herein relate to analyzing antibodies, but it is to be understood that these techniques can be applied to other proteins as well. The proteins may be clinical candidates although they are not so limited.

Disclosed are methods of predicting antibody aggregation. This aggregation prediction tool can be used early in the drug development process to enable ranking and selection of lead antibodies with reduced risk of aggregation.

Protein aggregation, a commonly encountered problem during biopharmaceutical development, has the potential to occur at different stages of the manufacturing and development processes, such as during fermentation, purification, formulation, fill-finish and storage. Aggregation potentially effects not only the manufacturing process, but also the target product profile, product efficacy, delivery and, critically, patient safety. Protein aggregates have been reported to contribute to cases of immune reactions in patients.

These aggregates can manifest themselves as reversible oligomers, subvisible or visible particles, or as precipitates. The protein aggregation process is driven by a number of factors, including amino acid composition and sequence, environmental factors such as pH, concentration, buffers/excipients and shear-forces during processes used for protein production, as well as final formulation and storage conditions.

In some aspects, the aggregation prediction can be used in combination with other *in silico* prediction tools to screen and select antibodies of interest. For example, the disclosed aggregation prediction model can be combined with the disclosed viscosity prediction model or with a known immunogenicity or degradation prediction tool. The combination of these tools allows for selection of one or more antibodies with a reduced risk of aggregation, viscosity, degradation, and/or immunogenicity to progress to in vitro expression and characterization.

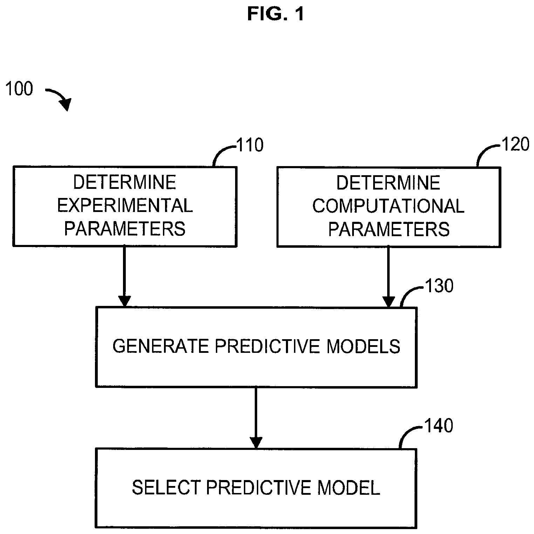

In an embodiment, shown in **FIG. 1,** a method **100** for generating a predictive model to assist in therapeutic screening, ranking, and/or selection is described. At **110,** experimental parameters may be determined. The experimental parameters may relate to, for example, protein yield, viscosity, aggregation, chemical stability (e.g., susceptibility to degradation through oxidation, deamidation), formulability, and/or immunogenicity.

Experimental parameters may be determined from experimental data. Experimental data can be, for example, data produced by a measurement, test method, experimental design, and/or quasi-experimental design. In clinical research any data produced are the result of a clinical trial. Experimental data may be qualitative or quantitative, each being appropriate for different investigations. Experimental data may comprise values for experimental parameters obtained through conducting one or more experiments associated with an antibody.

In an embodiment, experimental parameters associated with viscosity may be determined. In some aspects, the techniques for measuring viscosity measure how a sample reacts to flow, speed, and time. For example, a capillary viscometer can be used which measures the amount of time it takes a sample to pass through a tube. Similar to using a capillary viscometer, the Zahn cup method can be used wherein a small hole is placed in the bottom of a cup and the time it takes for a sample to pass through the hole is measured. The falling sphere viscometer technique can also be used to measure viscosity in which a sphere of known density is dropped into the sample and the time it takes for the sphere to fall to a specified point is recorded. In some aspects, a vibrational viscometer is used to measure the damping of an oscillating electromechanical resonator immersed in a sample. The rotational viscometer technique can also be used and it measures the torque required to turn an object in a sample as a function of that sample's viscosity.

In an embodiment, experimental parameters associated with antibody aggregation may be determined. Experimental parameters associated with antibody aggregation can be performed using any known protein aggregation technique. For example, biochemical assays for measuring aggregation include, but are not limited to, ultracentrifugation, size-exclusion chromatography, gel electrophoresis, dynamic light scattering or turbidity measurements. Many of these techniques take into account the size difference between a protein monomer and aggregates. Fluorescence based assays can also be used wherein a fluorophore increases its fluorescence yield in the presence of protein aggregates.

In an embodiment, experimental parameters associated with protein yield can be performed using techniques known in the art. Protein concentration is similar to protein yield but establishes how much protein is in a particular volume of solution. Protein concentration is most often determined using a spectrophotometer. Once the protein concentration is determined, the protein yield can be determined. Thus, if a sample has a protein concentration of 5mg/ml then if the protein yield results in 100ml then the total protein yield is 500mg.

In an embodiment, experimental parameters associated with antibody-antigen docking can be performed using any known techniques. The 'gold standard' for obtaining this data is by experimentally determining the 3D structure of the antibody-antigen complex using X-ray crystallography. Other structural methods such as cryo-electron microscopy (cryoEM) or nuclear magnetic resonance (NMR) can be used but the size of the complexes makes it challenging for the latter. These experimental data showing possible binding between an antibody and its antigen and can provide different conformational changes that can occur upon binding.

In an embodiment, experimental parameters associated with immunogenicity may be determined. Immunogenicity of a therapeutic antibody can cause detrimental side effects. Immunogenicity can be experimentally determined using animal experiments. An antibody can be administered to an animal (such as a mouse or a rabbit) and then at different time points the sera from the animal can be tested for an immune response (particularly T cell and B cell responses) to the antibody. In most cases, the lower the immunogenicity, the better option the therapeutic antibody becomes. In some aspects, the immunogenicity of an antibody can be altered by humanizing the antibody.

In an embodiment, experimental parameters associated with chemical stability may be determined. Chemical stability can be an important attribute of a therapeutic protein, specifically an antibody. In most instances, the more likely an antibody is to degrade, the less desirable it likely is as a therapeutic. The most common way for chemical stability to be experimentally determined is using gel electrophoresis. Pulse-chase assays can also be used. pH, temperature, and proteases can all be factors in chemical stability. Therefore, minor formulation changes can affect chemical stability.

At **120,** computational parameters may be determined. The computational parameters may be determined by computational analysis and/or simulation. Computational parameters may be determined from computationally-derived data. Computationally-derived data can be, for example, data produced by sequence analysis, antibody numbering, full FV region modeling, Ab-specific side chain prediction, antibody specific loop prediction, side chain prediction, *ab initio* loop prediction, CDR canonical structure prediction, VH/VL orientation, paratope prediction, protein contact prediction, Ab-specific epitope prediction, Ab-specific docking, unspecific docking, structure prediction, homology modeling, protein-protein docking simulation, molecular dynamics simulation, and the like. Experimental data may comprise values for experimental parameter obtained through computational analysis associated with an antibody.

In an embodiment, computational parameters may be determined through antibody numbering. Antibody sequences may be mapped onto a standardized reference framework. Raw nucleotide sequences of variable regions can be translated into amino acids by aligning them to germline sequences, thus identifying the V, D and J regions. This can be achieved by programs such as IgBLAST or IMGT V-Ques and multiple other tools aimed at processing raw antibody data. Similarities between antibody amino acid sequences further allow for the creation of a standardized reference framework, or numbering scheme, giving each variable region amino acid an identifier. The numbering schemes contextualize each position within the structure of an antibody, allowing for rapid delineation of CDR and framework regions. Antibody numbering may be a first step in computational antibody analysis such as homology modeling.

In an embodiment, computational parameters may be determined through antibody modeling. Structural antibody modeling creates a 3D structure from the antibody sequence, based on existing knowledge of antibody structures in particular and protein structures in general. The high degree of antibody sequence and structure conservation in the framework region and the five canonical loops leads to an overall high accuracy of antibody homology modeling. Antibody modeling generally involves selection of a suitable framework template that can harbor the CDR loops. This may be achieved by finding close sequence matches to the H and L chains in available databases. The relative orientation of the VH and VL domains are determined, which influences the shape of the paratope. The CDR loops are then modeled. Antibody-specific knowledge-based approaches can be used to predict CDR loops according to a template. If there is no suitable template, as can be often the case with CDRH3, more computationally expensive *ab initio* approaches can be employed that generate a large set of novel loops and selecting the best loop model. The side-chains are then built and refined. Protein-generic and/or antibody-focused approaches may be employed. The final antibody model can be further refined by optimizing the energetic packing of the molecule. Various modeling tools may be used, such as Biovia from Accelrys (https://www.3dsbiovia.com/), SmrtMolAntibody from Macromoltek (https://www.macromoltek.com/), MOE from CCG (https://www.chemcomp.com/) and BioLuminate from Schrodinger Inc. (https://www.schrodinger.com/products/bioluminate). Modeling tools can produce a model of the entire antibody $F_V$ with an accuracy of 1.1 Å Root Mean Square Deviation (RMSD) on average, with the most challenging region being the CDRH3, which is modeled to >5 Å RMSD in some targets. Such results typically cannot rival the accuracy of experimentally derived structures, but a model with 1.0 Å RMSD, can be used as a rapid proxy to delineate structural features of the molecule. Modeled structures can be used at the select surface exposed paratope residues for mutations or to characterize the binding with respect to the cognate epitope. Accurate structural information can be used to assess various developability indicators, such as hydrophobicity that rely on accurate models of the molecular surface of the paratope and epitope.

In an embodiment, computational parameters associated with residue charge may be determined through antibody homology modeling. Full antibody and/or Fab (antigen-binding fragment) homology models may be constructed via modeling software using protein data bank (PDB) crystal structures as templates. In an embodiment, full antibody and/or Fab homology models may be constructed to determine computational parameters for protein viscosity and/or protein aggregation propensity. As described herein, full antibody and/or Fab homology models may also be used in molecular dynamics simulations to determine computational parameters. The energies of antibody structures may be determined based on the homology model and then minimized through geometry optimization. The antibody structures may be protonated followed by determinations of computational parameters such as charges on residues and an average dipole moment.

The charges on one or more regions of an antibody ($Z_{VL}$, $Z_{VH}$, $Z_{CL}$, $Z_{CH1}$, $Z_{Hinge}$, $Z_{CH2}$, $Z_{CH3}$, $Z_{Total}$ in full antibody models and $Z_{VL}$, $Z_{VH}$, $Z_{CL}$, $Z_{CH1}$, $Z_{Total}$ in Fab models), including variable and constant regions for both light and heavy chains, may be determined as computational parameters. In an embodiment, each residue's net charge may be adjusted by considering a relative solvent accessible surface (SAS) of that residue within the homology model of the corresponding antibody. In an embodiment, an inbuilt algorithm within the discovery studio software can be used to determine the total exposed surface area of every amino acid. In this approach, the charge on each residue may be multiplied by a weighting factor calculated using the SAS of the residue relative to the total SAS of either full antibody or Fab depending on which model was being used. For example, in the variable light chain, the adjusted charge for each residue may be calculated using equation 3, and the total SAS adjusted charge for this region may be calculated using equation 4. These SAS adjusted charges may be labeled as $Z_{VL}^*, Z_{VH}^*, Z_{CL}^*, Z_{CH1}^*, Z_{Hinge}^*, Z_{CH2}^*, Z_{CH3}^*, Z_{Total}^*$ in full models and $Z_{VL}^*, Z_{VH}^*, Z_{CL}^*, Z_{CH1}^*, Z_{Total}^*$ in Fab models.

$$Z_{VL_i}^* = \frac{(SAS)_i}{\sum_{j=1}^{n}(SAS)_j} \times Z_{VL_i} \qquad \text{(Eq. 3)}$$

where $i$ = any residue in the variable light ($VL$) chain, and $n$ = the number of residues in the full or Fab model of a specified antibody.

$$Z_{VL}^* = \sum_{i=1}^{m} Z_{VL_i}^* \qquad \text{(Eq. 4)}$$

where $m$ = the number of residues in the variable light ($VL$) chain.

In an embodiment, full antibody and/or Fab homology models may be used to determine a hydrophobicity index (HI) as a computational parameter. The HI of the variable fragment (Fv) may be determined as $HI = -(\Sigma\, n_iE_i/\, \Sigma\, n_jE_j)$, where $i$ represents the hydrophobic amino acids, e.g., A, C, F, G, I, L, M, P, V, W, and Y, and $j$ represents the hydrophilic amino acids, e.g., D, E, H, K, N, Q, R, S, and T; n is the number of each amino acid, and $E$ is the Eisenberg scale value of each amino acid. In an embodiment, full antibody and/or Fab homology models may be used to determine an average dipole moment as a computational parameter. The average dipole moment of full and Fab models can be determined from protonated structures.

In an embodiment, full antibody homology models, Fab homology models, and/or antibody sequence data, may be used to determine an isoelectric point (pI). In an embodiment, full antibody and/or Fab homology models may be used to determine a per-atom aggregation propensity (AP) score. The AP score may be determined, for example, based on CHARMM forcefield and SAS patches of exposed hydrophobic residues in a radius of 10 Å. A total aggregation score for each antibody may be determined as a sum of aggregation scores of all residues in the either full antibody homology models or Fab homology models. An example of computational parameters that may be determined based on antibody homology models and/or Fab homology models is shown in Table 1.

| Table 1 |
|---|
| $Z_{VL}$ (charge on the $V_L$ region) |
| $Z_{VH}$ (charge on the $V_H$ region) |
| $Z_{CL}$ (charge on the $C_L$ region) |
| $Z_{CH1}$ (charge on the $C_H1$ region) |
| $Z_{Hinge}$ (charge on the hinge region) |
| $Z_{CH2}$ (charge on the $C_H2$ region) |
| $Z_{CH3}$ (charge on the $C_H3$ region) |
| $Z_{mAb}$ (total charge) |
| $Z_{VL}^*$ (solvent accessible surface, SAS, adjusted charge on the $V_L$ region) |
| $Z_{VH}^*$ (SAS adjusted charge on the $V_H$ region) |
| $Z_{CL}^*$ (SAS adjusted charge on the $C_L$ region) |
| $Z_{CH1}^*$ (SAS adjusted charge on the $C_H1$ region) |
| $Z_{Hinge}^*$ (SAS adjusted charge on the hinge region) |

(continued)

| Table 1 |
| --- |
| $Z^*_{CH2}$ (SAS adjusted charge on the $C_H2$ region) |
| $Z^*_{CH3}$ (SAS adjusted charge on the $C_H3$ region) |
| **HI** (hydrophobicity index) |
| **D$_{mAb}$** or **D$_{Fab}$** (total dipole moment) |
| **PI$_{Sequence}$** (sequence-based pI) |
| **pI$_{Structure}$** (structure-based pI) |
| **AP** (aggregation propensity predicted by Chennamsetty) |
| **RMSD** (root mean square deviation of conformational change) |

In an embodiment, computational parameters may be determined based on Molecular Dynamics (MD) simulations. MD simulations may be utilized to include the conformational changes of a Fab region associated with aggregation propensity. The atoms of Fab structures may be assigned forcefield parameters by matching each residue to its template structurally. These structures may be explicitly solvated in a truncated octahedral box of TIP3P water molecules. The counterions, $Na^+$ and $Cl^-$, may added to the explicitly solvated system to neutralize the system. In each simulation, the energy of system may be minimized with a steepest descent algorithm, followed by additional minimization with Adopted Basis Newton-Raphson (ABNR) minimization to remove large strains in the system. The systems may be heated gradually under a constant volume (NVT) and simulated at constant temperature and pressure. The particle mesh Ewald (PME) approach may be used to determine long-range electrostatics using a cutoff distance for van der Waals interactions. The SHAKE algorithm may be used in each simulation to constrain bond lengths to all hydrogen atoms. Simulations may be performed for each system, differing only on the initial distribution of velocities, to allow scrutiny of the reproducibility of the results. The trajectories, time varying atomic coordinates, of each simulation may be captured. In an embodiment, a backbone root mean square deviations (RMSD) of conformational structures relative to the initial structure after rigid-body alignment in each simulation may then be determined as a computational parameter, a descriptor of conformational stability.

In an embodiment, computational parameters may be determined through interface prediction and antibody-antigen docking. Computational methods may be employed to predict antibody-antigen contact surfaces. The computational methods may, for example, predict the paratope, the epitope, or the entire antibody-antigen complex. About half of the 40-50 residues in the CDRs are in direct contact with the antigen, forming the paratope. Statistical approaches such as Antibody i-Patch assign a score to each residue with respect to its propensity to be part of the paratope, with high-scoring residues offering potential candidates for mutagenesis. Since not all paratope residues are constrained to the CDRs, positions in the framework region that might contribute to antigen recognition can be computationally identified.

Computational methods for epitope prediction can be divided into predictors of linear epitopes, which focus on identifying contiguous stretches of primary amino acid sequence, and conformational epitope predictors, which aim to identify the 3D configuration of the epitope. Paratope and epitope prediction can offer useful information on antibody-antigen recognition, but these methods do not provide information about the specific interactions involved in antibody-antigen binding. This issue is addressed by antibody-antigen docking, a specialized application of the broader field of molecular docking. Molecular docking predicts the biological complex starting from the unbound proteins. It typically involves two steps; a sampling step, during which thousands of possible complex conformations are generated and a scoring step, where the conformations are ranked according to a specific scoring function to discriminate models that are closer to the native conformation.

In an embodiment, computational parameters may be determined through assessment of "humanness" of the therapeutic via sequence analysis. A large proportion of currently developed antibodies are discovered by animal immunizations. Molecules raised in animals, such as mice, carry the risk of inducing an immunological response in humans in the form of anti-drug antibodies (ADAs). To avoid such issues, animal-derived antibodies undergo a process called humanization. During this process the CDRs from the (typically) mice-derived antibodies are grafted onto human frameworks, or alternatively, the mice-derived frameworks are engineered to resemble human ones. Traditionally, humanization involves comparing the animal-derived sequence with approximately 1000 human germline sequences before selecting the appropriate template. Germline sequences however only offer a limited view of overall mutational antibody diversity, which can be addressed by computational humanization, comparing the animal-derived therapeutic to the distribution of amino acids in human antibody sequences. In an embodiment, computational methods may be employed that compare a query therapeutic sequence to a set of recombined variable region sequences that serve as a reference in humanization. In an embodiment, computational methods may be employed that assess the "humanness" of the query therapeutic sequence by determining how close the amino acid content of the query therapeutic sequence is to a human amino acid distribution.

In an embodiment, computational parameters may be determined through computational prediction of immune epitopes and ADAs generated against biotherapeutics. The generation of immune responses against a biotherapeutic requires multiple steps beyond reproducing human antibody sequence diversity. Humanized and even fully human antibodies can elicit immune responses among the patients receiving such treatments and generate ADAs against them. Generation of ADAs is a multi-factorial issue and may depend, for example, upon patient genetic background, disease history, protein aggregates in the therapeutic, and other degradants. A component of ADA generation is the binding of short biotherapeutic-derived peptide fragments to major histocompatibility complex class II (MHC II) molecules. Accordingly, computational methods may be used to identify potential MHC I and MHC II binding T-cell epitopes as well as conformational B-cell epitope and T-cell epitopes.

In an embodiment, computational parameters related to biophysical properties of a therapeutic may be determined. For example, biophysical properties such as colloidal stability of the antibody solution, concentration dependent viscosity behaviors, and physicochemical degradation. Solubility avoids aggregation that can potentially lead to loss of activity, degradation of antibodies, or immunogenicity. From a general perspective, protein aggregation has two aspects, mechanistic and kinetic. Mechanistic aspects focus on protein instability and on identifying potential APRs, mainly hydrophobic patches on the protein surface, which can potentially nucleate aggregation. Computational methods may be used to predict APRs in biotherapeutics according to sequence analysis, for example, the presence of multiple well defined aggregation prone motifs (often located in CDRs). These CDR-located APRs may contribute towards antigen binding. Additionally, sequence analysis may be used to identify aggregation rate enhancer and mitigatory mutations in proteins. Computational methods may be used to predict solubility. Sequence analysis may be used to determine the presence of one or more predictors of solubility and APRs in proteins. Computational methods may be used to predict hydrophobicity. Identification of hydrophobic regions may be performed using a homology model.

At **130,** one or more candidate predictive models may be determined. In an embodiment, the experimental parameters and the computational parameters may be analyzed to determine one or more predictive models that rely on computational parameters determined to significantly influence the experimental parameters. One or more computational approaches may be used to determine the one or more predictive models including, for example, adaptive context tree weighting, neural network, CART (classification and regression tree), projection pursuit regression, stepwise regression, linear regression, elastic net, multivalent models, MARS (multivariate adaptive regression splines), power law, primal graphical LASSO, ridge regression and generalized additive model (GAM).

In an embodiment, stepwise multiple regression (including forward selection, or backward elimination), forced entry, forced removal, and hierarchical multiple regression may be used to determine the one or more predictive models. For example, multiple regression analysis may be used to establish a relationship between all of the independent variables (e.g., experimental parameters), and the dependent variables (the computational parameters). The relationship establishes a relative influence of the independent variables. Then, forward selection (associated with stepwise regression) may be used to determine the relevance of the independent variables. Forward selection may begin with no independent variables in the equation (associated with multiple regression). The independent variable having the highest correlation, or influence, with the dependent variable may be added into the equation. The performance of the resulting predictive model may be determined using assessment techniques. The assessment technique, (e.g., "Goodness of Fit" analysis techniques), such as Akaike information criterion (AIC), R2, RMS, p-value, F ratios, standard error etc., may be used to establish performance characteristics of the relationships. For example, techniques such as R2, which establish the percent of variance in the dependent variable (e.g., the computational parameters), explained collectively by the independent variables (e.g. the experimental parameters). By using R2, for example, an assessment may be made regarding which relationship best explains the variance in the dependent variable in response to the independent variables. Techniques such as AIC, serve as an estimator of in-sample prediction error and thereby relative quality of the predictive models.

The forward selection process may be repeated, adding another independent variable (and associated coefficient) to the equation, and then assessing the equation. Once all the independent variables have been added, assessment metrics (e.g., AIC, R2) may be compared to determine which equation best described the relationship. The variables in the equation that best describes the relationship may be considered to be the most relevant variables, and the other variables may be ignored. For example, a determination may be made regarding which variable configuration resulted in the lowest AIC and/or a determination may be made regarding which variable configuration resulted in the highest R2, or noticeable improvements in R2. In another example, each time an independent variable is added, the relationship may be assessed to determine if there was a noticeable improvement (e.g., AIC decreased by an appreciable amount).

If the assessment metric did not change by a significant amount, then the process may be stopped, and the independent variables currently forming the relationship may be deemed to be the most relevant.

The backward elimination process (associated with stepwise regression) begins with all the independent variables in the equation and sequentially removes them, analogous to the forward process, to determine the desired relationship. For example, after establishing the relative influence of the independent variables, the least influential independent variable may be removed from the equation. If the resulting AIC is not significantly reduced, then the process may be repeated. In one embodiment, stepwise regression may be used when constructing the equation, or to prune the variables used in establishing the predictive model(s).

At **140,** a predictive model may be selected from the candidate predictive models generated at step **130.** In an embodiment, a validation technique such as Leave-One-Out Cross-Validation, or LOOCV, may be used to select the predictive model. LOOCV is a method whereby data a data point is is systematically excluded from the data set, after which its endpoint value is predicted by the relationship derived from the remaining data points (See, Cramer et al., Quant. Struct-Act. Relat. 7: 18-25, 1998, incorporated herein by reference). Cross-validation is useful for judging the reliability of relationships, especially where a validation data set is not available. The mean and standard deviation of errors of predicted LOOCV values from experimental values may be used as a criteria to compare and select a predictive model.

Once selected, the predictive model may be presented with novel computational parameters and make a prediction related to experimental parameters. For example, a predictive model may be trained according to experimental parameters associated with mAb solution viscosity and computational parameters associated with charge values of the mAb residues. The predictive model may be presented with computational parameters of the type the predictive model was trained with, and the predictive model will make a prediction associated with the experimental parameters the predictive model was trained with.

For example, a predictive model may be generated according to experimental parameters generated through viscosity measurements made of a plurality of mAb solutions. The viscosity measurements may obtained via use of a viscometer. Homology models may be generated computationally and used to determine charge values associated with residues of the mAbs. The charge values may be weighted based on whether the residues are determined to be surface-exposed residues. The charge values and/or the weighted charge values may be used as computational parameters. A predictive model may be generated according to the experimental parameters and the computational parameters. The predictive model may be configured to generate a score indicative of viscosity. A mAb on which the predictive model was not trained (e.g., an input mAb) may be modeled and charge values/weighted charge values generated.

The charge values and/or the weighted charge values may be provided to the predictive model which will generate a score indicative of the viscosity associated with the input mAb. For example, a predictive model may be generated according to experimental parameters generated through aggregation measurements made of a plurality of mAb solutions. The aggregation measurements may obtained via use of a dynamic light scattering. Homology models may be generated computationally and used to determine charge values associated with residues of the mAbs. The charge values may be weighted based on whether the residues are determined to be surface-exposed residues. The charge values and/or the weighted charge values may be used as computational parameters. A predictive model may be generated according to the experimental parameters and the computational parameters. The predictive model may be configured to generate a score indicative of aggregation. A mAb on which the predictive model was not trained (e.g., an input mAb) may be modeled and charge values/weighted charge values generated. The charge values and/or the weighted charge values may be provided to the predictive model which will generate a score indicative of the aggregation associated with the input mAb.

In some aspects, after providing, to the optimal predictive model, the computationally-derived data; and determining, based on the optimal predictive model, a viscosity score associated with the query mAb, one can adjust, based on the viscosity score, an appropriate formulation composition or protein engineering strategy to mitigate specific challenges with the drug candidate in development, for example, adjusting an amount of viscosity reducer of a solution associated with the query mAb. In some aspects, the same can be performed for an aggregation score in addition or instead of the viscosity score. Various formulation developments or protein engineering strategies can be designed in case high viscosity and aggregation scores are calculated for a mAb of interest. In some aspects, high aggregation and viscosity scores can indicate the presence of intermolecular interactions that can be driven by a combination of colloidal and conformational interactions. Various generally recognized as safe (GRAS) excipients are known to stabilize against colloidal and conformational instabilities and a combination of such excipients can be then be utilized to stabilize mAb structure and reduce viscosity. In some aspects, a high viscosity score and a low aggregation score can indicate that intermolecular interactions are transient and are primarily driven by colloidal interactions. Again, a range of GRAS excipients are known to reduce electrostatic and hydrophobic interactions between mAbs in solution. In some aspects, protein engineering can also be used to swap out specific amino acids that are responsible for such interactions. In some aspects, a high aggregation score and a low viscosity score may indicate a primarily conformational destabilization driven aggregation. Excipients such as sucrose, various diols and salts have shown to stabilize protein structure conformationally and can be used in such an event.

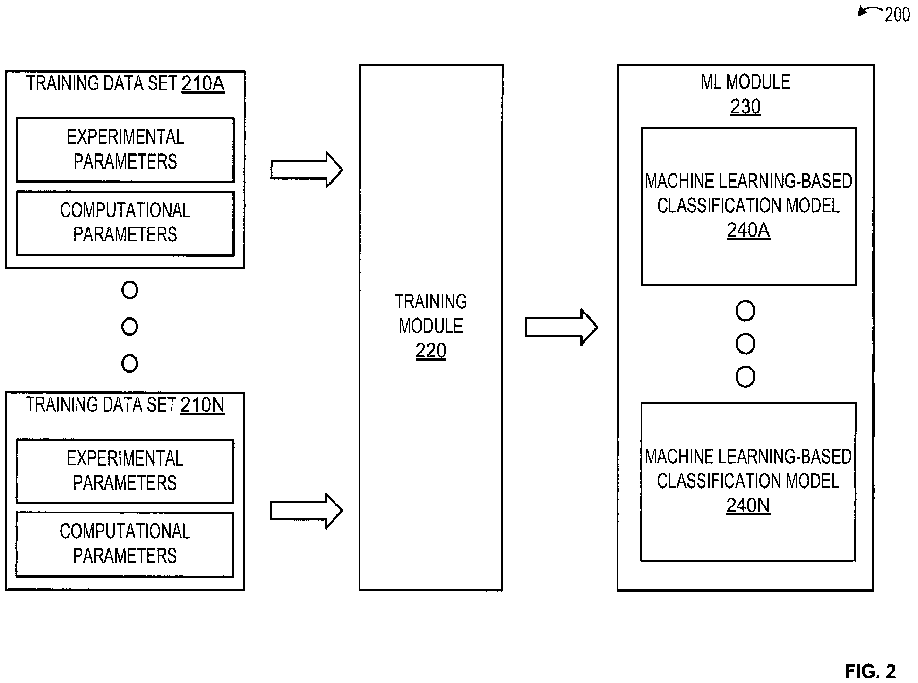

Turning now to **FIG. 2,** additional methods are described for generating a predictive model. The methods described may use machine learning ("ML") techniques to train, based on an analysis of one or more training data sets **210** by a training module **220**, at least one ML module **230** that is configured to predict a protein viscosity score and/or a protein aggregation score for a given antibody.

The training data set **210** may comprise experimental parameters associated with direct measurement of antibody solution viscosity and/or antibody aggregation. The experimental parameters are associated with computational parameters associated with the corresponding antibody. The computational parameters may be associated with charge values of resides on the corresponding antibody determined via computational modeling. For example, the measurement of viscosity of a first mAb solution may be associated with the charge values of the first mAb. Such data may be derived in whole or in part from experimental data and/or computationally-derived data as described herein.

A subset of the experimental parameters associated with computational parameters may be randomly assigned to the training data set **210** or to a testing data set. In some implementations, the assignment of data to a training data set or a testing data set may not be completely random. In this case, one or more criteria may be used during the assignment. In general, any suitable method may be used to assign the data to the training or testing data sets, while ensuring that the distributions of yes and no labels are somewhat similar in the training data set and the testing data set.

The training module **220** may train the ML module **230** by extracting a feature set from the computational parameters (e.g., labeled with experimental parameters) in the training data set **210** according to one or more feature selection techniques. The training module **220** may train the ML module **230** by extracting a feature set from the training data set **210** that includes statistically significant features.

The training module **220** may extract a feature set from the training data set **210** in a variety of ways. The training module **220** may perform feature extraction multiple times, each time using a different feature-extraction technique. In an example, the feature sets generated using the different techniques may each be used to generate different machine learning-based classification models **240.** For example, the feature set with the highest quality metrics may be selected for use in training. The training module **220** may use the feature set(s) to build one or more machine learning-based classification models **240A-240N** that are configured to indicate a computed viscosity and/or a computed aggregation score for a new mAb (e.g., with an unknown viscosity and/or an unknown aggregation).

The training data set **210** may be analyzed to determine any dependencies, associations, and/or correlations between features and the experimental parameters in the training data set **210**. The identified correlations may have the form of a list of features. The term "feature," as used herein, may refer to any characteristic of an item of data that may be used to determine whether the item of data falls within one or more specific categories. By way of example, the features described herein may comprise one or more of: $Z_{VL}$ (charge on the $V_L$ region), $Z_{VH}$ (charge on the $V_H$ region), $Z_{CL}$ (charge on the $C_L$ region), $Z_{CH1}$ (charge on the $C_H1$ region), $Z_{Hinge}$ (charge on the hinge region), $Z_{CH2}$ (charge on the $C_H2$ region), $Z_{CH3}$ (charge on the $C_H3$ region), $Z_{mAb}$ (total charge), $Z_{VL}^{*}$ (solvent accessible surface, SAS, adjusted charge on the $V_L$ region), $Z_{VH}^{*}$ (SAS adjusted charge on the $V_H$ region), $Z_{CL}^{*}$ (SAS adjusted charge on the $C_L$ region), $Z_{CH1}^{*}$ (SAS adjusted charge on the $C_H1$ region), $Z_{Hinge}^{*}$ (SAS adjusted charge on the hinge region), $Z_{CH2}^{*}$ (SAS adjusted charge on the $C_H2$ region), $Z_{CH3}^{*}$ (SAS adjusted charge on the $C_H3$ region), **HI** (hydrophobicity index), $D_{mAb}$ or $D_{Fab}$ (total dipole moment), $pI_{Sequence}$ (sequence-based pI), $pI_{Structure}$ (structure-based pI), **AP** (predicted aggregation propensity), and/or **RMSD** (root mean square deviation of conformational change).

A feature selection technique may comprise one or more feature selection rules. The one or more feature selection rules may comprise a feature occurrence rule. The feature occurrence rule may comprise determining which features in the training data set **210** occur over a threshold number of times and identifying those features that satisfy the threshold as features.

A single feature selection rule may be applied to select features or multiple feature selection rules may be applied to select features. The feature selection rules may be applied in a cascading fashion, with the feature selection rules being applied in a specific order and applied to the results of the previous rule. For example, the feature occurrence rule may be applied to the training data set **210** to generate a first list of features. A final list of features may be analyzed according to additional feature selection techniques to determine one or more feature groups (e.g., groups of features that may be used to predict viscosity and/or aggregation). Any suitable computational technique may be used to identify the feature groups using any feature selection technique such as filter, wrapper, and/or embedded methods. One or more feature groups may be selected according to a filter method. Filter methods include, for example, Pearson's correlation, linear discriminant analysis, analysis of variance (ANOVA), chi-square, combinations thereof, and the like.

The selection of features according to filter methods are independent of any machine learning algorithms. Instead, features may be selected on the basis of scores in various statistical tests for their correlation with the outcome variable.

As another example, one or more feature groups may be selected according to a wrapper method. A wrapper method may be configured to use a subset of features and train a machine learning model using the subset of features. Based on the inferences that drawn from a previous model, features may be added and/or deleted from the subset. Wrapper methods include, for example, forward feature selection, backward feature elimination, recursive feature elimination, combinations thereof, and the like. As an example, forward feature selection may be used to identify one or more feature groups. Forward feature selection is an iterative method that begins with no feature in the machine learning model. In each iteration, the feature which best improves the model is added until an addition of a new variable does not improve the performance of the machine learning model. As an example, backward elimination may be used to identify one or more feature groups. Backward elimination is an iterative method that begins with all features in the machine learning model. In each iteration, the least significant feature is removed until no improvement is observed on removal of features. Recursive feature elimination may be used to identify one or more feature groups. Recursive feature elimination is a greedy optimization algorithm which aims to find the best performing feature subset. Recursive feature elimination repeatedly creates models and keeps aside the best or the worst performing feature at each iteration. Recursive feature elimination constructs the next model with the features remaining until all the features are exhausted. Recursive feature elimination then ranks the features based on the order of their elimination.

As a further example, one or more feature groups may be selected according to an embedded method. Embedded methods combine the qualities of filter and wrapper methods. Embedded methods include, for example, Least Absolute Shrinkage and Selection Operator (LASSO) and ridge regression which implement penalization functions to reduce overfitting. For example, LASSO regression performs L1 regularization which adds a penalty equivalent to absolute value of the magnitude of coefficients and ridge regression performs L2 regularization which adds a penalty equivalent to square of the magnitude of coefficients.

After the training module **220** has generated a feature set(s), the training module **220** may generate a machine learning-based classification model **240** based on the feature set(s). A machine learning-based classification model may refer to a complex mathematical model for data classification that is generated using machine-learning techniques. In one example, the machine learning-based classification model **240** may include a map of support vectors that represent boundary features. By way of example, boundary features may be selected from, and/or represent the highest-ranked features in, a feature set.

The training module **220** may use the feature sets determined or extracted from the training data set **210** to build a machine learning-based classification model **240A-240N.** In some examples, the machine learning-based classification models **240A-240N** may be combined into a single machine learning-based classification model **240.** Similarly, the ML module **230** may represent a single classifier containing a single or a plurality of machine learning-based classification models **240** and/or multiple classifiers containing a single or a plurality of machine learning-based classification models **240.**

The features may be combined in a classification model trained using a machine learning approach such as discriminant analysis; decision tree; a nearest neighbor (NN) algorithm (e.g., k-NN models, replicator NN models, etc.); statistical algorithm (e.g., Bayesian networks, etc.); clustering algorithm (e.g., k-means, mean-shift, etc.); neural networks (e.g., reservoir networks, artificial neural networks, etc.); support vector machines (SVMs); logistic regression algorithms; linear regression algorithms; Markov models or chains; principal component analysis (PCA) (e.g., for linear models); multi-layer perceptron (MLP) ANNs (e.g., for non-linear models); replicating reservoir networks (e.g., for non-linear models, typically for time series); random forest classification; a combination thereof and/or the like. The resulting ML module **230** may comprise a decision rule or a mapping for each feature to determine viscosity and/or aggregation for an antibody.

In an embodiment, the training module **220** may train the machine learning-based classification models **240** as a convolutional neural network (CNN). The CNN comprises at least one convolutional feature layer and three fully connected layers leading to a final classification layer (softmax). The final classification layer may finally be applied to combine the outputs of the fully connected layers using softmax functions as is known in the art.

The feature(s) and the ML module **230** may be used to predict the viscosity and/or aggregation from the experimental parameters in the testing data set. In one example, the prediction result for each sequence includes a confidence level that corresponds to a likelihood or a probability that computational parameter of a mAb in the testing data set are associated with low/high viscosity and/or low/high aggregation. The confidence level may be a value between zero and one. In one example, when there are two statuses (e.g., low and high), the confidence level may correspond to a value $p$, which refers to a likelihood that a particular mAb belongs to the first status (e.g., low). In this case, the value $1-p$ may refer to a likelihood that the particular sequence belongs to the second status (e.g., high). In general, multiple confidence levels may be provided for each mAb in the testing data set and for each feature when there are more than two statuses. A top performing feature may be determined by comparing the result obtained for each test mAb with the known experiment parameters for each test mAb. In general, the top performing feature will have results that closely match the known yes/no promoter statuses. The top performing feature(s) may be used to predict the viscosity and/or aggregation status of a mAb.

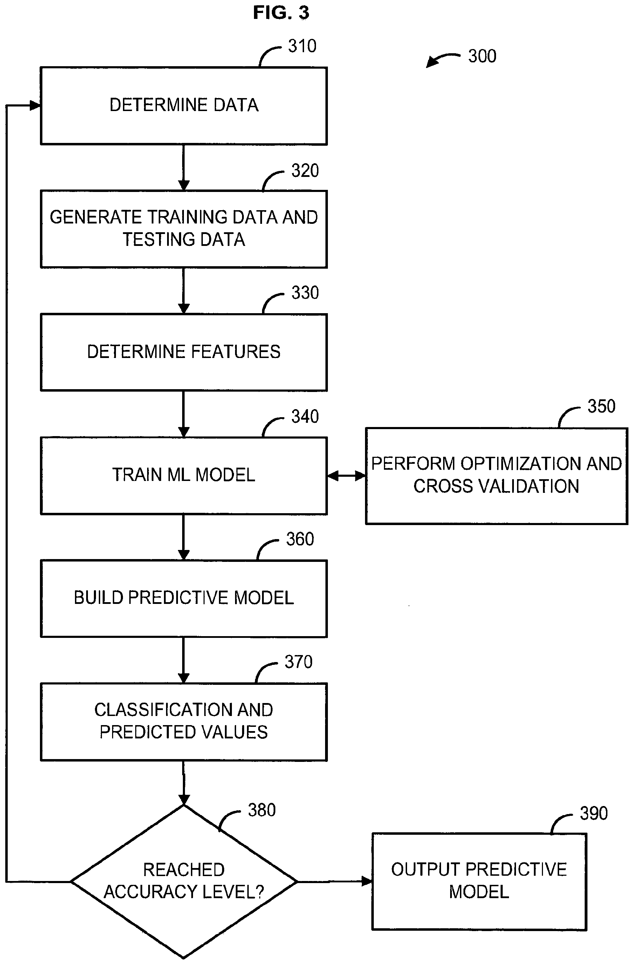

**FIG. 3** is a flowchart illustrating an example training method **300** for generating the ML module **230** using the training module **220**. The training module **220** can implement supervised, unsupervised, and/or semi-supervised (e.g., reinforcement based) machine learning-based classification models **240**. The method **300** illustrated in **FIG. 3** is an example of a supervised learning method; variations of this example of training method are discussed below, however, other training methods can be analogously implemented to train unsupervised and/or semi-supervised machine learning models.

The training method **300** may determine (e.g., access, receive, retrieve, etc.) data at step **310**. The data may comprise experimental parameters associated with direct measurement of antibody solution viscosity and/or antibody aggregation. The experimental parameters are associated with computational parameters associated with the corresponding antibody. The computational parameters may be associated with charge values of resides on the corresponding antibody determined via computational modeling.

The training method **300** may generate, at step **320**, a training data set and a testing data set. The training data set and the testing data set may be generated by randomly assigning computation parameters and associated experimental parameters to either the training data set or the testing data set. In some implementations, the assignment of computation parameters and associated experimental parameters as training or testing data may not be completely random. As an example, a majority of the computation parameters and associated experimental parameters may be used to generate the training data set. For example, 75% of the computation parameters and associated experimental parameters may be used to generate the training data set and 25% may be used to generate the testing data set. In another example, 80% of the computation parameters and associated experimental parameters may be used to generate the training data set and 20% may be used to generate the testing data set.

The training method **300** may determine (e.g., extract, select, etc.), at step **330**, one or more features that can be used by, for example, a classifier to differentiate among different classification of viscosity and/or aggregation status (e.g., low vs. high). As an example, the training method **300** may determine a set of features from the computation parameters and associated experimental parameters. In a further example, a set of features may be determined from data that is different than the computation parameters and associated experimental parameters in either the training data set or the testing data set. Such computation parameters and associated experimental parameters or other data may be used to determine an initial set of features, which may be further reduced using the training data set.

The training method **300** may train one or more machine learning models using the one or more features at step **340**. In one example, the machine learning models may be trained using supervised learning. In another example, other machine learning techniques may be employed, including unsupervised learning and semi-supervised. The machine learning models trained at **340** may be selected based on different criteria depending on the problem to be solved and/or data available in the training data set. For example, machine learning classifiers can suffer from different degrees of bias. Accordingly, more than one machine learning model can be trained at **340**, optimized, improved, and cross-validated at step **350**.

The training method **300** may select one or more machine learning models to build a predictive model at **360**. The predictive model may be evaluated using the testing data set. The predictive model may analyze the testing data set and generate predicted viscosity and/or aggregation statuses at step **370**. Predicted viscosity and/or aggregation statuses may be evaluated at step **380** to determine whether such values have achieved a desired accuracy level. Performance of the predictive model may be evaluated in a number of ways based on a number of true positives, false positives, true negatives, and/or false negatives classifications of the plurality of data points indicated by the predictive model.

For example, the false positives of the predictive model may refer to a number of times the predictive model incorrectly classified a mAb as a low viscosity or low aggregation that was in reality high viscosity or high aggregation. Conversely, the false negatives of the predictive model may refer to a number of times the machine learning model classified a mAb as high viscosity or high aggregation when, in fact, the mAb was low viscosity or low aggregation. True negatives and true positives may refer to a number of times the predictive model correctly classified one or more mAbs. Related to these measurements are the concepts of recall and precision. Generally, recall refers to a ratio of true positives to a sum of true positives and false negatives, which quantifies a sensitivity of the predictive model. Similarly, precision refers to a ratio of true positives a sum of true and false positives. When such a desired accuracy level is reached, the training phase ends and the predictive model (e.g., the ML module **230**) may be output at step **390**; when the desired accuracy level is not reached, however, then a subsequent iteration of the training method **300** may be performed starting at step **310** with variations such as, for example, considering a larger collection of sequence data.

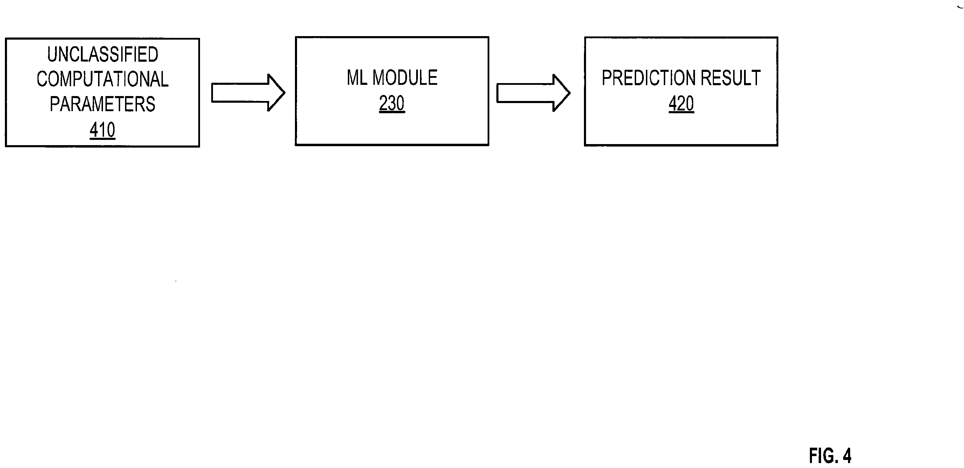

**FIG. 4** is an illustration of an exemplary process flow for using a machine learning-based classifier to determine whether a mAb is associated with low viscosity and/or low aggregation. As illustrated in **FIG. 4,** unclassified computational parameters of a mAb **410** may be provided as input to the ML module **230**. The ML module **230** may process the unclassified computational parameters of the mAb **410** using a machine learning-based classifier(s) to arrive at a prediction result **420.** The prediction result **420** may identify one or more characteristics of the unclassified computational parameters of the mAb **410.** For example, the classification result **420** may identify the viscosity and/or aggregation status of the unclassified computational parameters of the mAb **410** (e.g., whether or not the mAb has low/high viscosity and/or low/high aggregation).

**Examples**

**A. *In Silico* Predictive Models for Protein Solution Viscosity and Aggregation Propensity to Facilitate Drug Product Development**

In this study, two predictive models were developed: (1) a predictive model for solution viscosity by experimentally measuring viscosity values of a mix of 16 IgG1 and IgG4 antibodies and computational full-antibody homology modeling of the corresponding antibodies; and (2) a predictive model for aggregation propensity by experimentally measuring high-molecular-weight (HMW) species formation at accelerated thermal stress and computationally antigen-binding fragment (Fab) homology modeling and MD simulations of the corresponding Fab regions. The approach in this study is to adjust the charge of each residue in the homology models by a weight factor based on the relative solvent accessible surface (SAS) of exposed residue. With the aid of machine-learning algorithms, the computed electrostatic and hydrophobic parameters and conformational changes obtained from homology models and MD simulations, respectively, were assessed to build robust predictive models for protein solution viscosity and aggregation propensity.

**1. Methods**

**i. Protein Solution Viscosity Measurement**

Sixteen IgG1 and IgG4 antibody solutions, mAb1 to mAb16, formulated at 150 mg/mL protein concentration, 10 mM histidine buffer, pH 6.0 were prepared for viscosity measurement. Solution dynamic viscosity was measured at 20°C with an m-VROC viscometer (Rheosense, San Ramon, CA) at a rate of 100 $\mu$L/min with a shear rate of 1420 S$^{-1}$. Triplicate viscosity measurements were recorded over a duration of 100 seconds.

**ii. Osmotic Second Virial Coefficient ($B_{22}$) Measurement**

Antibody samples were diluted with corresponding buffer solutions to reach a final protein concertation of 10 mg/mL. Thereafter, samples were filtered through 0.22 $\mu$m Millex-GV syringe filter units (EMD Millipore, Billercia, MA). A fully automated composition-gradient multi-angle static light scattering (CG-MALS) instrument with a triple syringe-pump Calypso-II sample preparation and delivery unit (Wyatt Technology, Santa Barbara, CA) was used to measure light scattering at room temperature. A Mini Dawn Treos light scattering instrument (Wyatt Technology, Santa Barbara, CA), equipped with a 658 nm laser and an Optilab Rex refractive index detector (Wyatt Technology, Santa Barbara, CA), was used to measure both light scattering and protein concentration. Rayleigh ratio light scattering intensities were obtained over a protein concentration range of 2-8 mg/mL. Light scattering and protein concentration data were fit to equation 1, a virial expansion for non-ideal solutions, using Astra 6.1 software (Wyatt Technology, Santa Barbara, CA) to estimate $B_{22}$ values.

$$Kc/R_\theta = 1/M_W + 2B_{22}c \qquad \text{(Eq. 1)}$$

$R_0$ is the Rayleigh ratio, $M_w$ the molecular weight, and c is the protein concentration (mg/mL). $B_{22}$ signifies osmotic second virial coefficient, left unconstrained during data fitting. $B_{22}$, provides useful insights into intermolecular interactions between protein molecules in dilute solutions. A negative value of $B_{22}$ indicates that the overall interactions between protein molecules are attractive, while a positive value indicates that the overall interactions are repulsive. $K$ in equation 1 is the optical constant described by equation 2

$$K = \frac{4\pi^2 n^2 (dn/dc)^2}{N_A \lambda^4} \qquad \text{(Eq. 2)}$$

with *n* as the refractive index of the solvent (1.33), $N_A$ is Avogadro's number (mol$^{-1}$), *dn/dc* is the refractive index increment of the protein/solvent pair (0.185 mL/g), and $\lambda$ is the wavelength of the incident light in vacuum.

### iii. Diffusion Interaction Parameter ($K_D$) Measurement

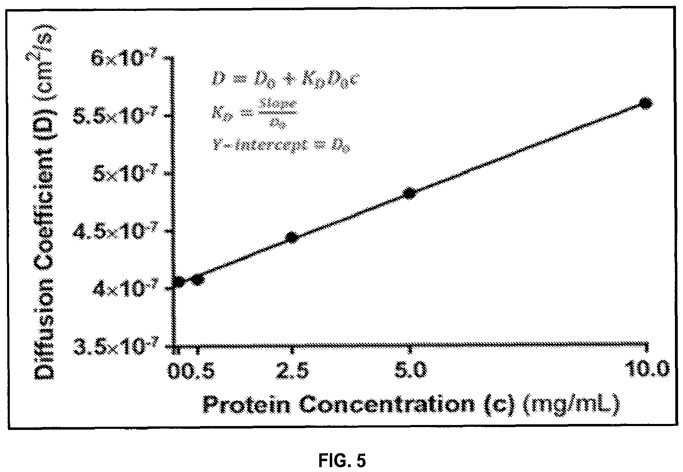

Antibody solutions were diluted with 10 mM histidine buffer (pH 6.0), to prepare samples of each mAb at 10, 5, 2.5, and 0.1 mg/mL protein concentrations. MAb1 was excluded from $K_D$ measurement because of limited material availability. The samples were centrifuged at 12,000 × g for 5 minutes before analysis to eliminate microbubbles in solution. Light scattering (DLS) was measured using the DynaPro Plate Reader (Wyatt Technology, Santa Barbara, CA). Fifteen runs of 15 second acquisitions were collected and averaged to determine the diffusion coefficient for each sample. The interaction parameter $K_D$ was calculated based on the following equation: $D = D_0 + K_D D_0 c$, where $D$ is the diffusion coefficient at a given protein concentration $c$, and $D_0$ represents the diffusion coefficient when c is close to 0. **FIG. 5** shows how $K_D$ was calculated based on fitting a line to the diffusion coefficients at various protein concentrations using mAb4 as an example.

### iv. Computational Homology Modeling

Full antibody and Fab homology models of sixteen IgG1 and IgG4 antibodies were constructed via BIOVIA Discovery Studio 2017 R2. The IgG1 and IgG4 antibodies were modeled using protein data bank (PDB) crystal structures of 1HZH and 5DK3, respectively as templates and their provided genetic sequences. Full antibody models were constructed to compute physical properties used later to develop a predictive model for protein viscosity. While, Fab models were used to compute physical properties and were ultimately used in MD simulations to develop a predictive model for aggregation propensity. The framework to model antibody structures are described thoroughly by Kemmish et al. The energies of antibody structures were then minimized through geometry optimization with CHARMM forcefield in 200 steps. The structures were protonated at pH 6.0 followed by calculation of charges on residues and the averaged dipole moment.

### v. Computed Parameters

The charges on all regions of the antibodies including variable and constant regions for both light and heavy chains ($Z_{VL}$, $Z_{VH}$, $Z_{CL}$, $Z_{CH1}$, $Z_{Hinge}$, $Z_{CH2}$, $Z_{CH3}$, $Z_{Total}$ in full antibody models and $Z_{VL}$, $Z_{VH}$, $Z_{CL}$, $Z_{CH1}$, $Z_{Total}$ in Fab models) were calculated using to the procedure described in the computational homology modeling section. Each residue's net charge was adjusted by considering the relative solvent accessible surface (SAS) of that residue within the homology model of the corresponding antibody. In this approach, the charge on each residue was multiplied by a weighting factor calculated using the SAS of the residue relative to the total SAS of either full antibody or Fab depending on which model was being used. For example, in the variable light chain, the adjusted charge for each residue was calculated using equation 3, and the total SAS adjusted charge for this region was calculated using equation 4. These SAS adjusted charges are labeled as:

$Z_{VL}^*, Z_{VH}^*, Z_{CL}^*, Z_{CH1}^*, Z_{Hinge}^*, Z_{CH2}^*, Z_{CH3}^*, Z_{Total}^*$ in full models and

$Z_{VL}^*, Z_{VH}^*, Z_{CL}^*, Z_{CH1}^*, Z_{Total}^*$ in Fab models.

$$Z_{VL_i}^* = \frac{(SAS)_i}{\sum_{j=1}^{n}(SAS)_j} \times Z_{VL_i} \qquad (Eq.\ 3)$$

where $i$ = any residue in the variable light ($VL$) chain, and $n$ = the number of residues in the full or Fab model of a specified antibody.

$$Z_{VL}^* = \sum_{i=1}^{m} Z_{VL_i}^* \qquad (Eq.\ 4)$$

where m = the number of residues in the variable light (VL) chain.

The hydrophobicity index (HI) of the variable fragment (Fv) was calculated using the method described by Sharma et al as $HI = -(\Sigma\ n_i E_i / \Sigma\ n_j E_j)$, where $i$ represents the hydrophobic amino acids, i.e., A, C, F, G, I, L, M, P, V, W, and Y, and $j$ represents the hydrophilic amino acids, i.e., D, E, H, K, N, Q, R, S, and T; n is the number of each amino acid, and $E$ is the Eisenberg scale value of each amino acid. As mentioned above, the averaged dipole moment of full and Fab models was computed at protonated structures at pH 6.0. The isoelectric point (pI) of antibodies used in this study were computed from both structural homology models and the sequence. The per-atom aggregation propensity (AP) scores are calculated based on CHARMM forcefield and SAS patches of exposed hydrophobic residues in a radius of 10 Å. The total aggregation score for each mAb is calculated as a sum of aggregation scores of all residues in the either full or Fab models. A comprehensive list of computed parameters in full antibody and Fab models can be found in Table 1 and the values of these computed parameters for each mAb are listed in the tables of **FIG. 6A, FIG. 6B** for full antibody and **FIG. 7** for Fab models.

**Table 1.** The correlation coefficient (R) obtained from a linear regression between experimentally measured values of viscosity and each computed parameter in the full antibody model; and between experimentally measured rate of high-molecular-weight species formation per day (%ΔHMW/Day) and each computed parameter in the Fab model..

| Computed parameters | Correlation coefficient (R) | |
|---|---|---|
| | Full antibody model | Fab model |
| $Z_{VL}$ (charge on the $V_L$ region) | 0.45 | -0.52 |
| $Z_{VH}$ (charge on the $V_H$ region) | -0.51 | 0.27 |
| $Z_{CL}$ (charge on the $C_L$ region) | 0.27 | -0.22 |
| $Z_{CH1}$ (charge on the $C_H1$ region) | -0.68 | 0.72 |
| $Z_{Hinge}$ (charge on the hinge region) | 0.33 | NA |
| $Z_{CH2}$ (charge on the $C_H2$ region) | -0.57 | NA |
| $Z_{CH3}$ (charge on the $C_H3$ region) | -0.42 | NA |
| $Z_{mAb}$ (total charge) | -0.55 | 0.11 |
| $Z^*_{VL}$ (solvent accessible surface, SAS, adjusted charge on the $V_L$ region) | 0.44 | -0.33 |
| $Z^*_{VH}$ (SAS adjusted charge on the $V_H$ region) | -0.48 | 0.20 |
| $Z^*_{CL}$ (SAS adjusted charge on the $C_L$ region) | -0.34 | 0.06 |
| $Z^*_{CH1}$ (SAS adjusted charge on the $C_H1$ region) | -0.36 | 0.01 |
| $Z^*_{Hinge}$ (SAS adjusted charge on the hinge region) | 0.54 | NA |
| $Z^*_{CH2}$ (SAS adjusted charge on the $C_H2$ region) | -0.19 | NA |
| $Z^*_{CH3}$ (SAS adjusted charge on the $C_H3$ region) | -0.57 | NA |
| HI (hydrophobicity index) | -0.43 | 0.14 |
| $D_{mAb}$ or $D_{Fab}$ (total dipole moment) | 0.26 | -0.29 |
| $pI_{Sequence}$ (sequence-based pI) | -0.30 | 0.29 |
| $pI_{Structure}$ (structure-based pI) | -0.44 | -0.14 |
| AP (aggregation propensity predicted by Chennamsetty) | -0.05 | 0.27 |
| RMSD (root mean square deviation of conformational change) | 0.71 | -0.59 |

**i. Mathematical Predictive Modeling of Protein Viscosity**

The experimental viscosity values and computed parameters were fed into a stepwise regression algorithm as dependent and independent variables, respectively. This algorithm generates new linear regression models by adding significant parameters and removing insignificant parameters from the list of parameters and compare the generated models based on Akaike information criterion (AIC); an estimator of the relative quality of statistical models for a given dataset based on the number of estimated parameters in a model and the maximum value of the likelihood function of that model. The combination of parameters in Wilkinson notation to generate more models were also considered. The outcome would be possible predictive models that can be compared together based on their $R^2$, p-value, and the mean and standard deviation of errors of predicted viscosity scores (PVSs) from the experimental values. Furthermore, the models were assessed through the leave-one-out cross validation (LOOCV) to ensure the effectiveness of the models in predicting unseen datasets. LOOCV was performed by excluding one mAb from the training set at a time to assess the robustness of the model in predicting the excluded data point. This analysis was repeated 16 times for each model by leaving out the computed and experimental data on a different mAb each time. The mean and standard deviation of errors of predicted LOOCV viscosity scores from the experimental values were used as another criteria to compare models. Scripts were developed in the R environment to facilitate building predictive models in a high-throughput automated pipeline.

**ii. Accelerated Thermal Stress Stability; Measurement of Aggregation Kinetics**

Accelerated stability studies are regularly performed during formulation development in pharmaceutical companies. For this study, the effect of thermal stress on overall stability was evaluated by incubating 14 mAb samples used in the viscosity study for 0, 7, 14, and 28 days at 40 °C and 75% relative humidity. Because of limited availability of mAb 9 and mAb16 materials, these 2 candidates were excluded from the current dataset for development of a predictive model for aggregation propensity. Size-exclusion chromatography (SEC) was used to measure amount of high-molecular-weight (HMW) species formation. The relative percentage of HMW formations for 7, 14, and 28 days, %ΔHMW, was calculated by comparing to the 0 day. Furthermore, the rate of %ΔHMW formation per day was calculated based on %ΔHMW of 28-day data points divided by 28.

**iii. Molecular Dynamics (MD) Simulations**

To include the conformational changes of Fab region in the predictive models of aggregation propensity, MD simulations of Fab models were utilized. The atoms in the 14 antibody minimized Fab structures were assigned to CHARMM36 forcefield parameters by matching each residue to its template structurally. These structures were then explicitly solvated in a truncated octahedral box of TIP3P water molecules. The counterions, $Na^+$ and $Cl^-$, were added to the explicitly solvated system to neutralize the system at an ionic concentration of 0.145 mol/L. In each simulation, the energy of system was first minimized with 1000 steps of steepest descent algorithm, followed by a further 2000-step minimization with ABNR to remove the large strains in the system. The systems were gradually heated up from 50.0 to 300.0 K in 4ps, in intervals of 50 K, under a constant volume (NVT) ensemble with a time step of 2.0 fs. Then, each system was further equilibrated for 10 ps with 2.0 fs time step at target temperature of 300.0 K under an isotropic pressure of 1.0 bar. Finally, each system was simulated for 2000 ps (i.e., 2.0 ns) with a time step of 2.0 fs at constant temperature of 300.0 K and pressure of 1.0 bar.

The particle mesh Ewald (PME) approach was employed for long-range electrostatics using a 10 Å cutoff distance for van der Waals interactions. The SHAKE algorithm was applied in each simulation to constrain bond lengths to all hydrogen atoms to allow a 2.0 fs time step. Three simulations of 2.0 ns, an overall of 6.0 ns, were performed for each system, differing only on the initial distribution of velocities, to allow scrutiny of the reproducibility of the results. The trajectories, time varying atomic coordinates, of each simulation were captured every 1.0 ps (i.e., an overall of 2000 conformations for each simulation). The backbone root mean square deviations (RMSD) of conformational structures relative to the initial structure after rigid-body alignment in each simulation were computed as a descriptor of conformational stability.

**iv. Mathematical Predictive Modeling of Aggregation Propensity**

As mentioned previously, protein aggregation is the collective effect of colloidal stability (i.e., intermolecular interactions) and conformational stability (i.e., protein structural changes). To build predictive models for aggregation propensity, the experimental rate of HMW formation (i.e., %ΔHMW/day), the physical computed parameters for Fab models (Table 1) as colloidal stability descriptors, and the averaged RMSDs in MD simulations as conformational stability descriptors were used in the same protocol described for the viscosity section. In summary, the stepwise regression algorithm was used to generate the most statistically significant models that correlate the colloidal and conformational computed parameters to the measured aggregation kinetics. These models were compared to each other according to the AIC number, p-value, $R^2$, adjusted $R^2$, the mean and standard deviation of absolute error, the performance on LOOCV, and the structural symmetry of antibody structures.

### 2. Results

### i. Protein Solution Viscosity

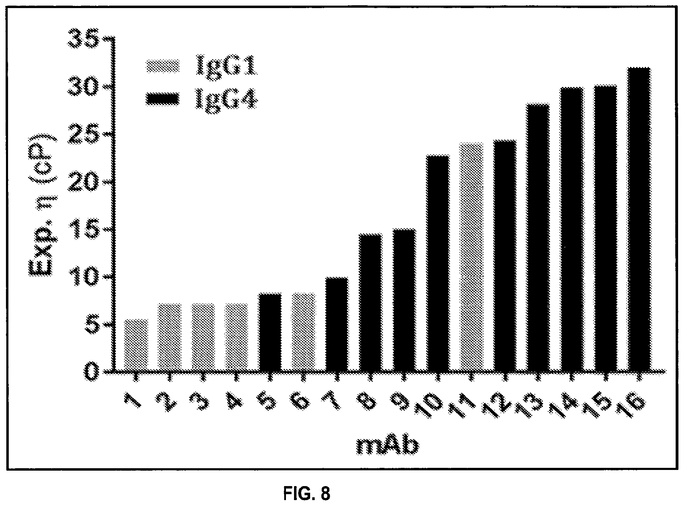

The viscosity values were measured for 16 mAbs at a protein concentration of 150 mg/mL. Overall, the viscosity values show a broad distribution ranging from 5.5 to 32.0 cP (Table 2 and **FIG. 8**). Based on the dataset, the IgG1 antibodies tend to show lower viscosity values when compared to IgG4 candidates (**FIG. 8**).

**Table 2.** The measured viscosity and computed predicted viscosity score (PVS) values for 16 mAbs used in this study. The viscosity values were measured at a formulation of 150 mg/mL protein concentration, 10 mM histidine buffer, and pH 6.0. The PVS values were calculated based on equation 5. The absolute errors were calculated between PVS and measured viscosity values. The PVS and absolute errors in the leave-one-out cross validation (LOOCV) are also shown.

| Antibody | Isotype | Exp. $\eta$ (cP), 150 mg/mL | PVS (all mAbs) | Absolute Error (all mAbs) | PVS (LOOCV) | Absolute Error (LOOCV) |
|---|---|---|---|---|---|---|
| mAb1 | IgG1 | 5.5 $\pm$ 0.0 | 5.8 | 0.3 | 6.1 | 0.6 |
| mAb2 | IgG1 | 7.1 $\pm$ 0.1 | 7.8 | 0.7 | 9.2 | 2.1 |
| mAb3 | IgG1 | 7.1 $\pm$ 0.1 | 10.8 | 3.7 | 12.0 | 4.9 |
| mAb4 | IgG1 | 7.1 $\pm$ 0.1 | 9.2 | 2.1 | 10.7 | 3.6 |
| mAb5 | IgG4 | 8.2 $\pm$ 0.0 | 10.5 | 2.3 | 11.8 | 3.6 |
| mAb6 | IgG1 | 8.2 $\pm$ 0.0 | 6.9 | 1.3 | 4.0 | 4.2 |
| mAb7 | IgG4 | 9.9 $\pm$ 0.1 | 6.2 | 3.7 | 3.9 | 6.0 |
| mAb8 | IgG4 | 14.5 $\pm$ 0.0 | 17.6 | 3.1 | 19.3 | 4.8 |
| mAb9 | IgG4 | 15.0 $\pm$ 0.2 | 16.4 | 1.4 | 18.5 | 3.5 |
| mAb10 | IgG4 | 22.7 $\pm$ 0.2 | 26.3 | 3.6 | 29.5 | 6.8 |
| mAb11 | IgG1 | 24.0 $\pm$ 0.2 | 20.4 | 3.6 | 16.6 | 7.4 |
| mAb12 | IgG4 | 24.3 $\pm$ 0.2 | 22.2 | 2.1 | 21.2 | 3.1 |
| mAb13 | IgG4 | 28.1 $\pm$ 0.1 | 32.0 | 3.9 | 34.6 | 6.5 |
| mAb14 | IgG4 | 29.9 $\pm$ 0.2 | 26.7 | 3.2 | 24.8 | 5.1 |
| mAb15 | IgG4 | 30.1 $\pm$ 0.8 | 22.2 | 7.9 | 20.8 | 9.3 |
| mAb16 | IgG4 | 32.0 $\pm$ 0.2 | 32.8 | 0.8 | 33.7 | 1.7 |

### ii. $B_{22}$ and $K_D$ Show Strong Correlation to Each Other, But Not to Viscosity

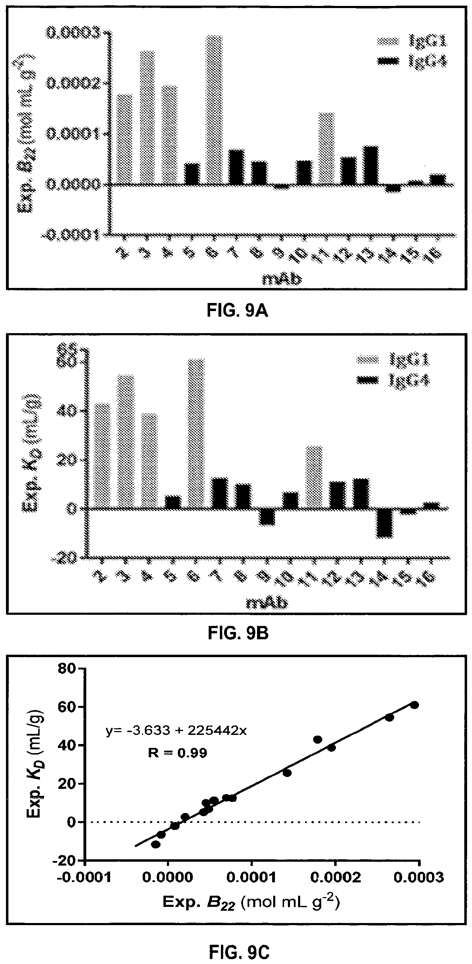

The osmotic second virial coefficient ($B_{22}$) and diffusion interaction parameter ($K_D$) were measured for 16 and 15 mAbs, respectively. For the current dataset, the $B_{22}$ values vary between -1.461 $\times 10^{-05}$ and 2.939$\times 10^{-04}$ mol ml g$^{-2}$ and the $K_D$ values vary between -11.604 and 61.114 mL/g (**FIGs. 9A-9C**). Based on the dataset, the IgG1 antibodies tend to show higher positive $B_{22}$ and $K_D$ values when compared to IgG4 candidates. The $B_{22}$ and $K_D$ values are strongly correlated to each other for the current dataset with a linear correlation coefficient (R) of 0.99 (**FIGs. 9A-9C**). This observation agrees with previously published work by other researchers in the field.

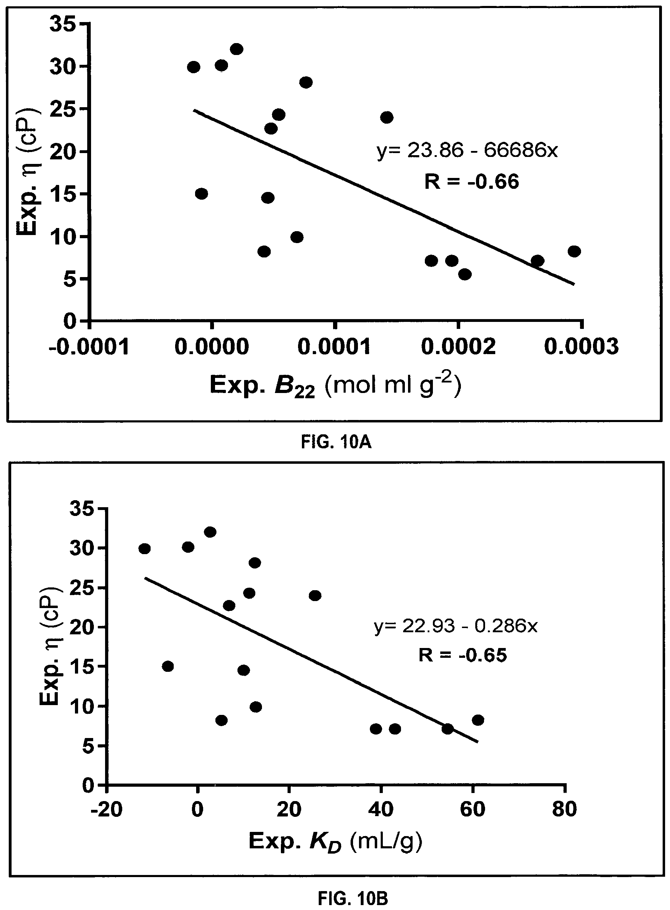

Both $B_{22}$ and $K_D$ measurements are measures of pairwise interactions mostly prevailed at dilute concentrations. However, as the concentration rises, the higher-order interactions involving multiple molecules also contribute significantly toward solution viscosity. Therefore, the measured $B_{22}$ and $K_D$ values at dilute solutions are not a direct measure of protein-protein interactions at high concentrations. However, there is a debate in the literature concerning the validity of using B22 and Kd values at dilute concentrations as a predictor of viscosity values at high protein concentrations. To assess this, the linear correlation between either $B_{22}$ or $K_D$ and measured viscosity values were determined (**FIGs. 10A-10B**). Based on the dataset, although there is a directional decreasing trend (i.e., negative correlation) between either $B_{22}$ or $K_D$ and viscosity values, there is no strong correlation (**FIGs. 10A-10B**). Hence, $B_{22}$ and $K_D$ are insufficient to predict protein solution viscosities when mAb concentrations rise.

### iii. Each Single Selected Computed Parameter Contributes to Predict Overall Viscosity Value

The linear correlation coefficient (R) values obtained from regression lines between measured viscosity values and computed parameters from the full-antibody homology models of 16 mAbs are shown in Table 1. Moreover, Figs. 11A-11C show the plots of linear relationship between the experimental viscosity values and the computed parameters. The correlation R values vary between -0.68 and 0.54. The computed parameters used in the final predictive model (i.e., $Z_{VL}^*$, $Z_{CL}^*$, $Z_{Hinge}^*$, $Z_{CH2}^*$, $Z_{CH3}^*$, and HI) were selected based on the stepwise protocol described in the mathematical modeling section and the structural symmetry of antibodies. While each one of these parameters contributes to predict overall viscosity value, no single computed parameter can predict the viscosity value by itself as observed by moderate R values. It was indeed expected as the nature of viscosity is a multivariable phenomenon involving intermolecular interactions defined by hydrophobic and electrostatic properties of various regions.

### iv. Predictive Model for Protein Viscosity: Predicted Viscosity Score (PVS)

The final predictive model for protein viscosity was selected based on the stepwise protocol and selected computed parameters as described in the previous sections. Predicted viscosity score (PVS) is a predictive model for protein viscosity considering the solvent accessible surface (SAS), adjusted charges on the $V_L$, $C_L$, hinge, $C_H2$, and $C_H3$ regions of a full antibody model and the hydrophobicity of the variable region (Eq. 5).

The constants $C_0$ to $C_6$ in the PVS model are shown in Table 3.

$$PVS = C_0 + C_1 \times Z_{VL}^* + C_2 \times Z_{CL}^* + C_3 \times Z_{Hinge}^* + C_4 \times Z_{CH2}^* + C_5 \times Z_{CH3}^* + C_6 \times HI$$

(Eq. 5)

**Table 3:** The constant coefficients in equation 5, the predicted viscosity score (PVS).

|  | **Estimate** | **Standard Error** |
|---|---|---|
| $C_0$ | $8.40500 \times 10^{+01}$ | $1.73900 \times 10^{+01}$ |
| $C_1$ | $9.02450 \times 10^{+02}$ | $2.04000 \times 10^{+02}$ |
| $C_2$ | $-3.6780 \times 10^{+02}$ | $4.68810 \times 10^{+02}$ |
| $C_3$ | $2.93309 \times 10^{+03}$ | $1.09175 \times 10^{+03}$ |
| $C_4$ | $-2.0760 \times 10^{+03}$ | $6.36320 \times 10^{+02}$ |
| $C_5$ | $-3.7017 \times 10^{+02}$ | $2.28880 \times 10^{+02}$ |
| $C_6$ | $-4.0510 \times 10^{+01}$ | $1.36200 \times 10^{+01}$ |

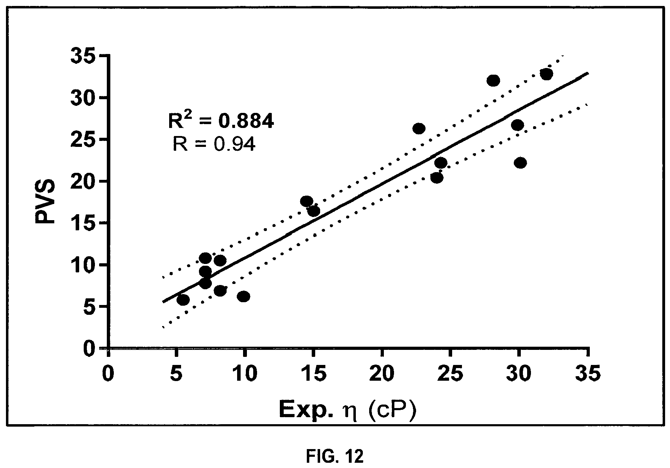

The PVS model has an $R^2$ of 0.884 and adjusted $R^2$ of 0.807. The correlation R value of 0.94 shows a strong correlation between PVS and the measured viscosity values in the current dataset; and the adjusted R value of 0.90 shows that this model fits the given dataset by considering the number of parameters in the model without a concern of over-fitting the data. The p-value of PVS model is 0.0009, indicating strong evidence against the null hypothesis that the PVS model is not capable of predicting viscosity values at 95% level of confidence (p-value < 0.05). The observed mean absolute error of 2.7 with a standard deviation of 1.8 between PVS and experimental values prove the efficacy of this model. The minimum and maximum residual error between PVS and measured viscosity is -7.9 and 3.9, respectively (Table 2). A linear regression line between the computed PVS values and the measured viscosity values shows that most of the data points lie in the 95% confidence interval (**FIG. 12**). More importantly, the PVS model performs well in the LOOCV analysis with a mean absolute error of 4.6 and a standard deviation of 2.3 between the LOOCV PVS and experimental viscosity values (Table 2). During the LOOCV, $R^2$ values ranged from 0.863 to 0.925 and the adjusted $R^2$ values ranged from 0.760 to 0.868. These results confirm that PVS represents a statistically significant predictive model between the selected computed parameters and the experimental viscosity values.

**v. Thermal Stress Stability Results**

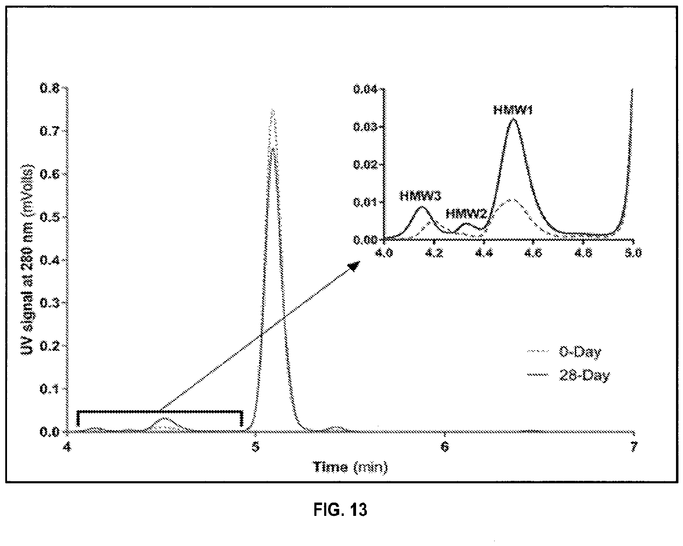

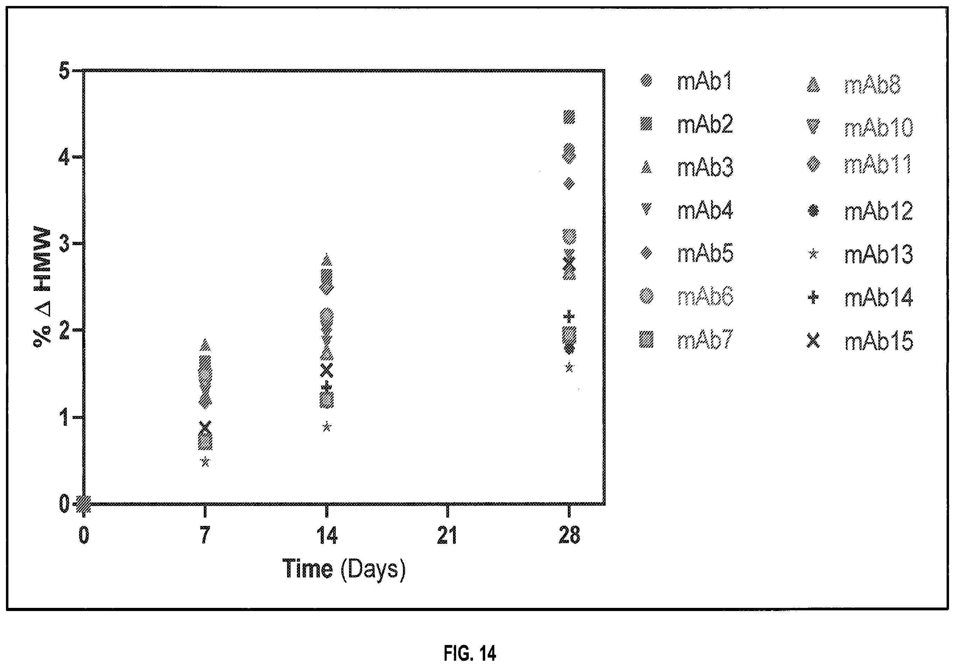

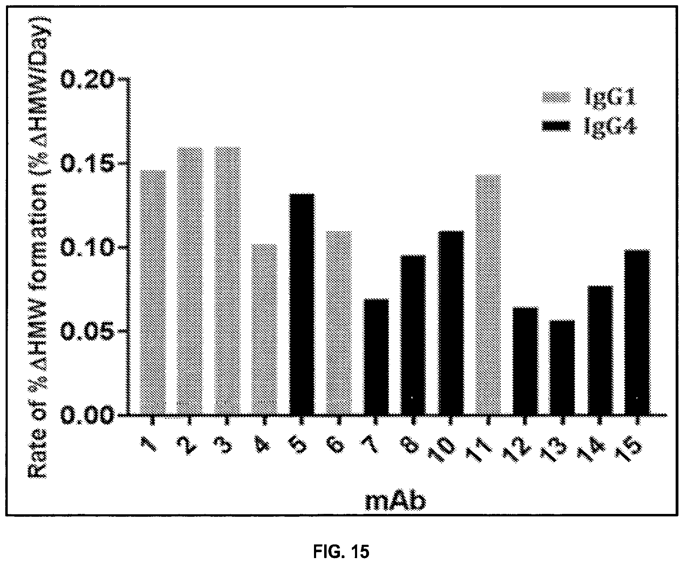

Fourteen mAbs were incubated at 40 °C and 75% relative humidity. **FIG. 13** shows a representative SEC signal for mAb3 over a period of time for 0-day and 28-day incubation and the increase in HMW peaks as a result of aggregate formations. The relative percentage of HMW formations for 7, 14, and 28 days compared to the 0 day, %ΔHMW, are shown in **FIG. 14** for 14 mAbs used in this study. As mAb samples incubated longer, more aggregates formed (**FIG. 14**). The rate of %ΔHMW formation per day, calculated based on %ΔHMW of 28-day data points divided by 28, range from 0.0564 to 0.1600 (Table 4 and **FIG. 15**). The IgG1 antibody molecules, tending to have lower viscosity values, tend to have higher %ΔHMW/day compared to IgG4 candidates on average for the current dataset (**FIG. 15**).

**Table 4.** The measured rate of high-molecular-weight species formation per day (%ΔHMW/Day) and computed predicted aggregation score (PAS) values for 14 mAbs used in this study. Because of limited availability of mAb 9 and mAb16 materials, these 2 candidates were excluded from the current dataset for development of a predictive model for aggregation propensity. The %ΔHMW/Day values were calculated after 28-day incubation at 40 °C and 75% relative humidity. The PAS values were calculated based on equation 6. The absolute errors were calculated between PAS and measured %ΔHMW/Day values. The PAS and absolute errors in the leave-one-out cross validation (LOOCV) are also shown.

| Antibody | Isotype | Exp. %ΔHMW/Da y | PAS (all mAbs) | Absolute Error (all mAbs) | PAS (LOOCV ) | Absolute Error (LOOCV) |
|---|---|---|---|---|---|---|
| mAb1 | IgG1 | 0.1461 | 0.1453 | 0.0008 | 0.1450 | 0.0011 |
| mAb2 | IgG1 | 0.1596 | 0.1627 | 0.0031 | 0.1647 | 0.0051 |
| mAb3 | IgG1 | 0.1600 | 0.1310 | 0.0290 | 0.1187 | 0.0413 |
| mAb4 | IgG1 | 0.1021 | 0.1097 | 0.0075 | 0.1160 | 0.0139 |
| mAb5 | IgG4 | 0.1321 | 0.1310 | 0.0012 | 0.1298 | 0.0023 |
| mAb6 | IgG1 | 0.1100 | 0.1080 | 0.0020 | 0.1066 | 0.0034 |
| mAb7 | IgG4 | 0.0696 | 0.0678 | 0.0018 | 0.0617 | 0.0080 |
| mAb8 | IgG4 | 0.0954 | 0.0872 | 0.0082 | 0.0827 | 0.0126 |
| mAb10 | IgG4 | 0.1100 | 0.1316 | 0.0216 | 0.1579 | 0.0479 |
| mAb11 | IgG1 | 0.1432 | 0.1370 | 0.0062 | 0.1318 | 0.0115 |
| mAb12 | IgG4 | 0.0643 | 0.0742 | 0.0099 | 0.0874 | 0.0231 |
| mAb13 | IgG4 | 0.0564 | 0.0593 | 0.0029 | 0.0663 | 0.0098 |
| mAb14 | IgG4 | 0.0771 | 0.0676 | 0.0095 | 0.0438 | 0.0333 |
| mAb15 | IgG4 | 0.0989 | 0.1125 | 0.0136 | 0.1282 | 0.0293 |

**vi. Further Validation of PVS**

To further test the predictability of the PVS model (Eq. 5), 4 IgG1 and IgG4 mAbs (Table 5) that were not part of the 16 mAbs used for developing the predictive model were assessed. The viscosity of these 4 mAbs were measured at the same formulation with the same protocol described earlier. The measured viscosity values range from 4.1 to 22.0 cP (Table 5). The structures of these 4 mAbs were modeled following the protocols described in this work. Hydrophobic and electrostatics parameters were computed (Table 5) for utilization in the PVS model. The absolute error between PVS and experimental viscosity values range from 2.8 to 5.6 (Table 5) showing that the PVS model is indeed capable of predicting viscosity values of mAbs not included in the training dataset.

**Table 5.**

| The computed parameters, measured viscosity values, and predicted viscosity scores (PVSs) for 4 mAbs that were not part of the 16 mAbs used for developing the predictive model for viscosity. $Z_{VL}^*$ $Z_{CL}^*$, $Z_{Hinge}^*$, $Z_{CH2}^*$, and $Z_{CH3}^*$ are solvent accessible surface adjusted charges on the $V_L$, $C_L$, hinge, and $C_H2$ regions, respectively and HI is the hydrophobicity index. The PVS values were calculated based on equation 5 and the viscosity values were measured at a formulation of 150 mg/mL protein concentration, 10 mM histidine buffer, and pH 6.0. The absolute errors were calculated between PVS and measured viscosity values. |
|---|

| Antibod y | Isotyp e | $Z_{VL}^*$ | $Z_{CL}^*$ | $Z_{Hinge}^*$ | $Z_{CH2}^*$ | $Z_{CH3}^*$ | HI | PVS | Exp. $\eta$ (cP), 150 mg/mL | Absolut e Error |
|---|---|---|---|---|---|---|---|---|---|---|
| mAb A | IgG1 | 0.019 3 | 0.001 6 | 0.0006 | 0.0201 | 0.0050 | 1.1 2 | 13.7 | 10.7 | 3.0 |
| mAb B | IgG1 | 0.014 0 | 0.001 8 | 0.0019 | 0.0145 | 0.0053 | 1.2 1 | 20.5 | 14.9 | 5.6 |
| mAb C | IgG4 | 0.013 6 | 0.000 0 | 0.0021 | 0.0198 | - 0.0094 | 1.1 2 | 19.5 | 22 | 2.5 |
| mAb D | IgG1 | 0.021 2 | 0.004 0 | - 0.0031 | 0.0172 | 0.0026 | 1.2 1 | 6.9 | 4.1 | 2.8 |

### vii. MD Simulation Results

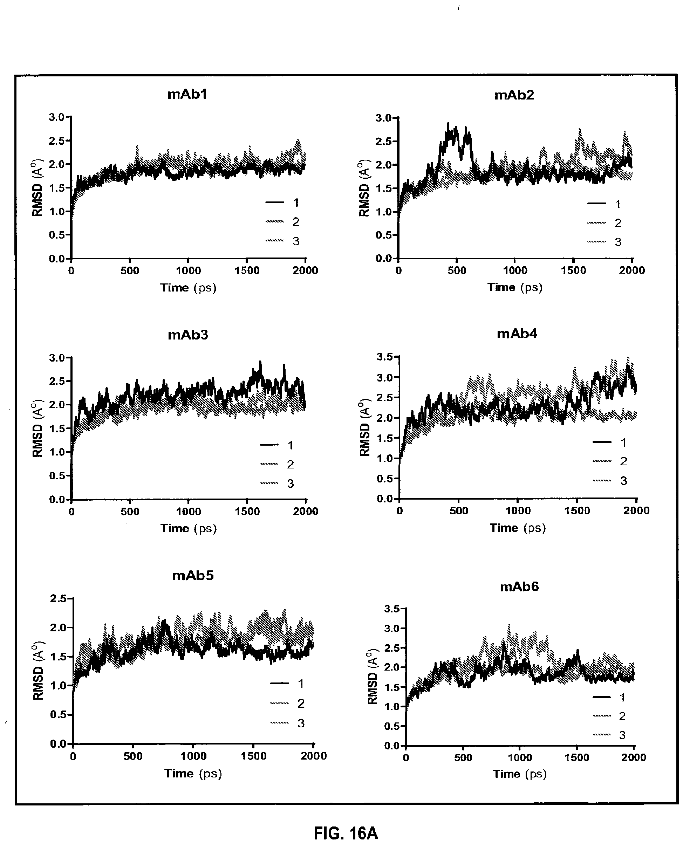

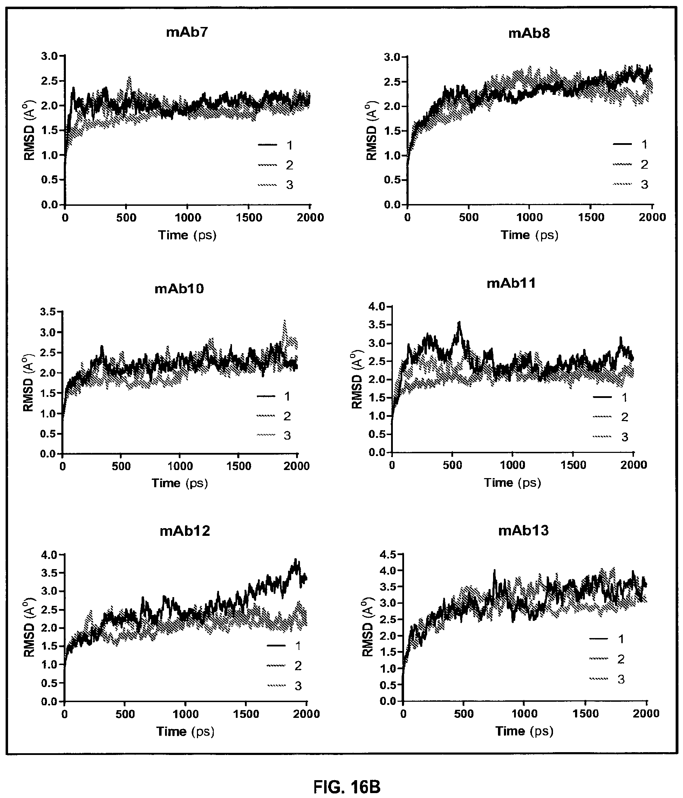

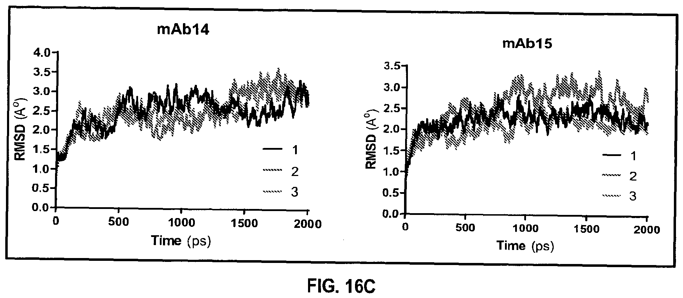

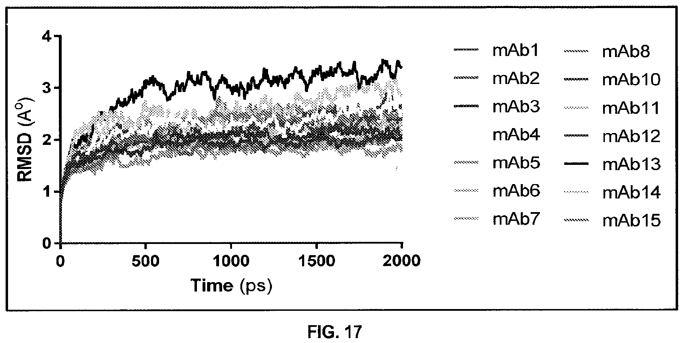

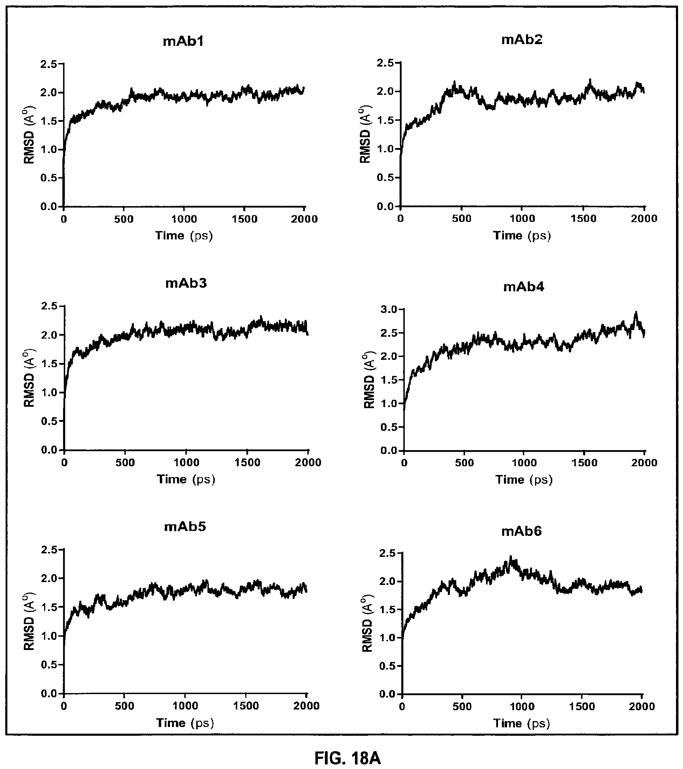

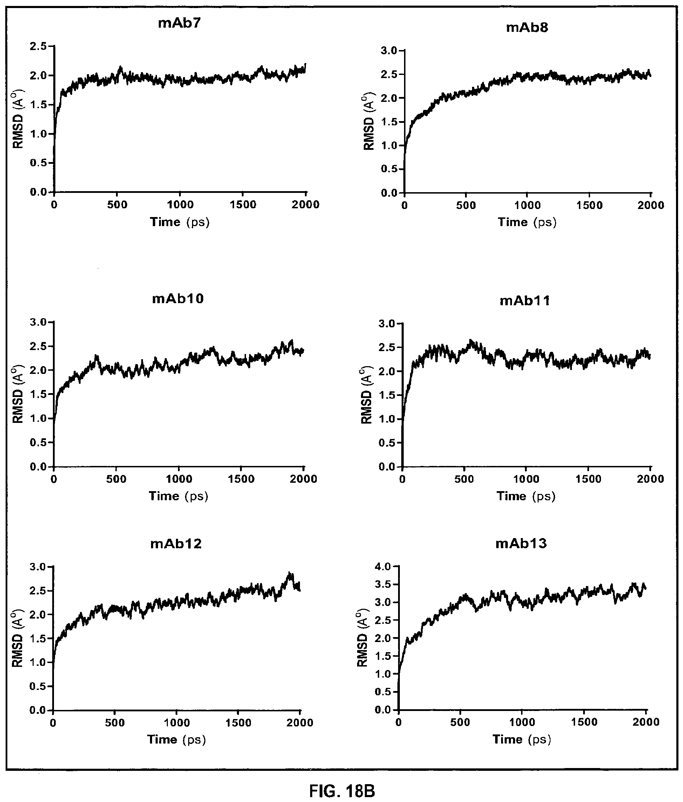

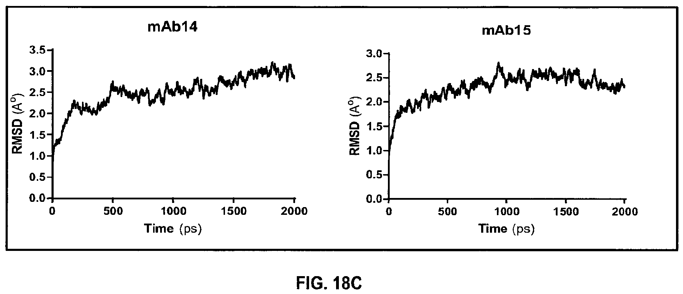

For each Fab model of 14 mAbs, three individual MD simulations were performed to assess the consistency of the observations. The RMSDs of conformational structures relative to the initial structure for each mAb are plotted over 2.0 ns time of simulation (**FIG. 16A-16C**) showing that overall, simulations for each mAb are reproducible. For each mAb, the RMSD values at each time point were averaged over three simulations and the averaged RMSDs are plotted against the 2.0 ns time of simulation in **FIG. 17**. Moreover, **FIG. 18A-18C** show the averaged RMSDs for each mAb in separate plots. The averaged RMSD values over 3 simulations for each mAb were averaged over the last 1.5 ns to obtain a single number as the average RMSD for each mAb. The average RMSDs range from 1.785 to 3.159 Å for the Fab region of 14 mAbs used in this study (**FIG. 7**).

### viii. Each Single Selected Computed Parameter Contributes to Predict Overall Aggregation Propensity

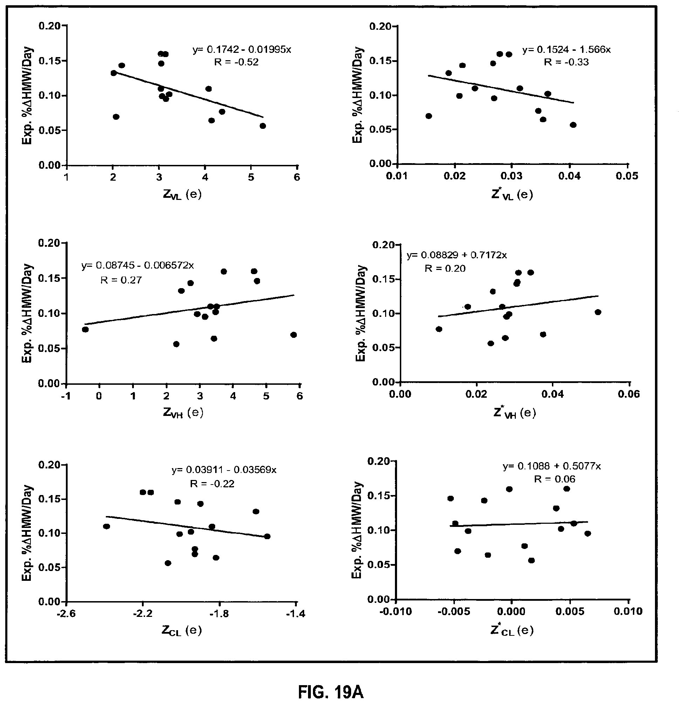

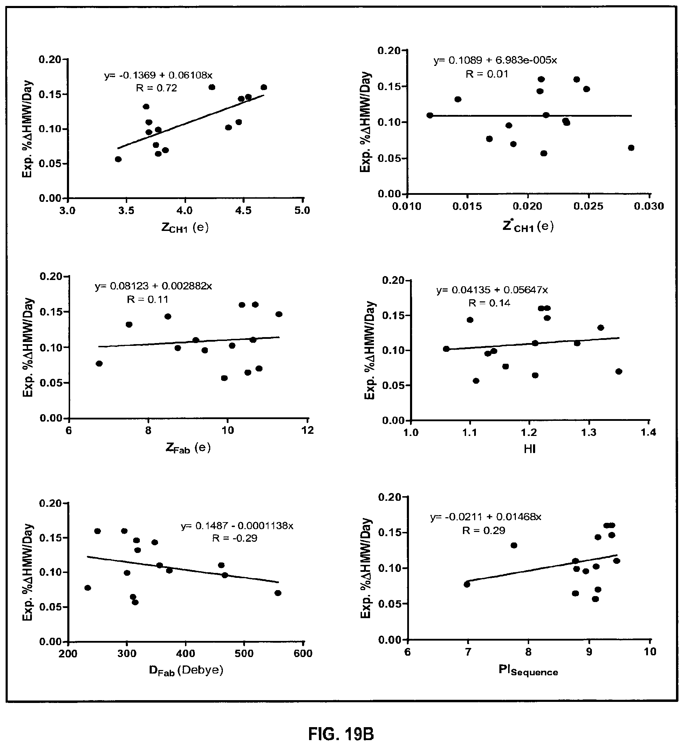

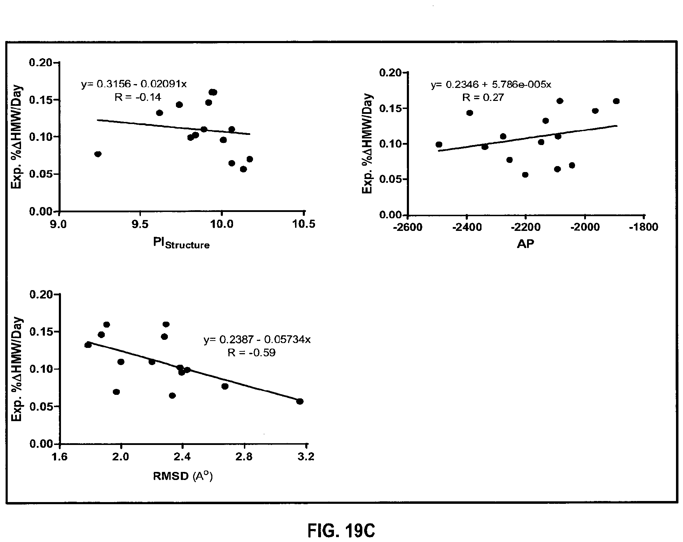

The computed parameters from the Fab homology models and the RMSD values from MD simulations are descriptors of colloidal and conformational stability, respectively. The linear correlation coefficient (R) values obtained from regression lines between measured %ΔHMW/Day values and computed parameters or RMSD values from MD simulations of 14 mAbs are shown in Table 1. Moreover, FIG. 19A-19C show the plots of the linear relationship between the experimental %ΔHMW/Day values and the computed parameters including RMSD. The correlation R values vary between -0.59 and 0.72. The computed parameters used in the final predictive model (i.e., $Z_{VL}^*$, $Z_{CH1}^*$, RMSD, HI, $D_{Fab}$, and $PI_{sequence}$) were selected based on the stepwise protocol described in the mathematical modeling section and the structural symmetry of the Fab region. While each one of these parameters contributes to predict an overall %ΔHMW/Day value, no single computed parameter can predict the %ΔHMW/Day value by itself as observed by moderate R values. This was expected as a result of the nature of aggregation being a multivariable phenomenon involving both colloidal and conformational stability terms.

### ix. Predictive Model for Aggregation Propensity: Predicted Aggregation Score (PAS)

The final predictive model for aggregation propensity was selected based on the stepwise protocol and selected computed parameters as described in the methods section. Predicted aggregation score (PAS) is a predictive model for protein aggregation kinetics including both colloidal and conformational computed descriptors of the Fab region. This model considers the SAS adjusted charges on $V_L$ and $C_{H1}$ regions, the averaged backbone RMSD of conformational changes relative to the initial structure, the hydrophobicity of the variable region, the dipole moment of the Fab region, and the isoelectric point of an antibody obtained from its sequence (Eq. 6). The constants $C_0$ to $C_6$ in the PAS model are shown in Table 6.

$$PAS = C_0 + C_1 \times Z_{VL}^* + C_2 \times Z_{CH1}^* + C_3 \times RMSD + C_4 \times HI + C_5 \times D_{Fab} + C_6 \times PI_{Sequence} \text{ (Eq. 6)}$$

**Table 6:** The constant coefficients in equation 6, the predicted aggregation score (PAS).

|  | Estimate | Standard Error |
|---|---|---|
| $C_0$ | $4.244 \times 10^{-01}$ | $1.547 \times 10^{-01}$ |
| $C_1$ | $-6.476 \times 10^{-01}$ | $9.244 \times 10^{-01}$ |
| $C_2$ | $-3.907 \times 10^{+00}$ | $1.480 \times 10^{+00}$ |
| $C_3$ | $-8.079 \times 10^{-02}$ | $1.991 \times 10^{-02}$ |
| $C_4$ | $-1.774 \times 10^{-01}$ | $7.944 \times 10^{-02}$ |
| $C_5$ | $-2.974 \times 10^{-04}$ | $6.308 \times 10^{-05}$ |
| $C_6$ | $3.187 \times 10^{-02}$ | $9.075 \times 10^{-03}$ |

The PAS model has an $R^2$ of 0.883 and adjusted $R^2$ of 0.782. The correlation R value of 0.94 shows a strong correlation between PAS and the measured %ΔHMW/Day values in the current dataset; and the adjusted R value of 0.88 shows that this model has well fitted the given dataset by considering the number of parameters in the model without a concern of over-fitting the data. The p-value of PAS model is 0.0057, indicating strong evidence against the null hypothesis that the PAS model is not capable of predicting %ΔHMW/Day values at 95% level of confidence (p-value < 0.05). The observed mean absolute error of 0.0084 with a standard deviation of 0.0083 between PAS and experimental values prove the efficacy of this model. The minimum and maximum residual error between PAS and measured %ΔHMW/Day is -0.0290 and 0.0216, respectively (Table 3).

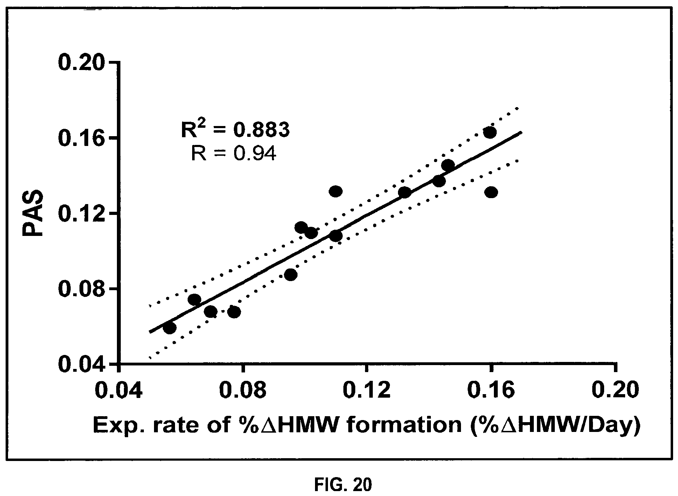

A linear regression line between the computed PAS values and the measured %ΔHMW/Day values shows that most of the data points lie in the 95% confidence interval (**FIG. 20**). More importantly, the PAS model performs well in the LOOCV analysis with a mean absolute error of 0.0173 and a standard deviation of 0.0151 between the LOOCV PVS and experimental %ΔHMW/Day values (Table 4). During the LOOCV, $R^2$ values ranged from 0.858 to 0.949 and the adjusted $R^2$ values ranged from 0.716 to 0.897. These results confirm that PAS represents a statistically significant predictive model between the selected colloidal and conformational computed parameters and the experimental %ΔHMW values.

## 3. Discussion

The production of monoclonal antibodies is increasing in the pipeline of biopharmaceutical companies. The trend of transition from IV to SC administrations raises challenges in formulation development for these candidates that require higher potency dosing due to viscosity and mAb aggregation issues. More robust methods should be developed to enable prediction of viscosity and aggregation propensity earlier in formulation development and drug discovery. The combination of *in silico* tools with experimental approaches are promising to resolve these challenges. Here, schemes to utilize the power of homology modeling and MD simulations were developed to generate predictive models for antibody solution viscosity and aggregation propensity. These models, PVS and PAS, compare different antibodies together in drug product development and even early discovery without the need for physical materials.

Compared to the similar predictive models in the literature for protein solution viscosity and aggregation propensity, the PVS and PAS models developed in this work show lower errors in prediction and are more reliable based on their $R^2$, adjusted $R^2$, p-value, absolute error values, and LOOCV analyses. The robustness of PVS and PAS models are acquired by taking advantage of novel computed parameters that adjust charge distributions relative to the SAS of each residue in different regions of an antibody and by considering the symmetry of its structure. In the PVS model, the electrostatics and hydrophobic computed parameters from antibody homology models are considered to reflect the intra- and inter-molecule interactions in a protein solution.

This is the first time that both colloidal and conformational stability parameters via all-atom MD simulations have been used in an aggregation propensity predictive model. Hence, the PAS model can predict the aggregation propensity more realistically by looking into stability in atomic details. It should be mentioned that the constant coefficients in the PVS and PAS predictive models developed for viscosity and aggregation propensity are specific to the buffer systems and the respective protein concentrations used in this study. However, the overall scheme and computed parameters can be extended to other buffer systems and protein concentrations.

The robustness and accuracy of predictive models in machine-learning algorithms and statistical methods depend on the number of data points in the training and validation datasets. However, the nature of experimental viscosity, aggregation measurements, and limited availability of physical materials restrict the ability of researchers in this field to obtain a large number of data points. Hence, all data points were used as the training dataset and the LOOCV analysis was performed to assess the predictability of models. The PVS and PAS models were developed based on viscosity and aggregation measurements of 16 and 14 mAbs, respectively. More data points from measurements taken at the same conditions can be added to the dataset developed for these models to improve the robustness and accuracy of these predictive models. The new data points can also be used as validation datasets to assess further predictability of PVS and PAS models. Moreover, the stepwise regression algorithm was utilized in this study to generate predictive models from computed parameters. Further statistical and machine-learning algorithms techniques such as least absolute shrinkage and selection operator (LASSO) regression and random forest regression can be explored to develop more robust models.

As mentioned earlier, to consider the conformational stabilities in the aggregation propensity model, the MD simulations were performed on the Fab region of antibodies. The described protocol in this work can be extended to perform MD simulations on full antibodies to generate conformational computed parameters for full antibody models. These parameters might improve the efficacy and reliability of the aggregation propensity model. Moreover, the full antibody all-atom and coarse-grained MD simulations can shed more light onto intra- and inter-molecular interactions of antibody molecules. As an example, Cloutier et al. analyzed the impact of excipients on aggregation and viscosity through all-atom MD simulations on three IgG1 mAbs. Kastelic et al. performed coarse-grained MD simulations to assess fragment antigen (Fab-Fab) or fragment crystallizable (Fab-Fc) binding interactions and suggested strategies to control the viscosities of antibody solutions through control of their binding sites.

In the current study, each mAb was solvated in a truncated octahedron water box to minimize the number of water atoms in favor of heavy computational simulations. Even with this scheme, each solvated full antibody or Fab model consists of around 275,000 or 51,000 atoms, respectively. Such large systems require heavy computational powers and time of simulations can be limited to available infrastructures. With the advancement of graphics processing units (GPUs), longer MD simulations on the Fab region and full antibodies are possible. Longer MD simulations might shed more light on the structural conformations and the inherent instability of antibodies in the solution and give us a better understanding of intra-and inter-molecular interactions. Furthermore, other schemes like the rectangular water boxes can be utilized to consider the interaction of more water molecules with a specified antibody. The box shape used in the simulations might have an impact on the dynamic behavior of proteins and computed properties.

Furthermore, all MD simulations in this study were performed in a water solution. This is the most common approach in the MD simulations of biologics as the most force fields are optimized for water interactions. However, performing MD simulations in a buffer solution environment similar to the final drug product can yield more relevant computed parameters to experimental viscosity and stability measurements. In the case of ranking antibodies, as long as all mAb candidates are treated in the same buffer system, they can be used in predictive models to be compared against each other.

### B. Model Validation

A set often (10) mAbs were used to conduct validation experiments for both PVS (predicted viscosity score) and PAS (predicted aggregation score) models and algorithms. The data statistical correlation between experimental data set and predicted scores was used to validate prediction algorithms for viscosity and aggregation.

### 1. Method

Experimental data for dynamic viscosity (cP) and % total aggregation at 40°C for 10 additional mAbs was used to validate prediction models. The experimental data was kept blinded from the user to remove any known and unknown biases. Predicted scores for viscosity and aggregation were then compared to experimental data and correlated using linear regression model. Any correlation score of above 0.75 is considered an acceptable correlation given the small size of dataset.

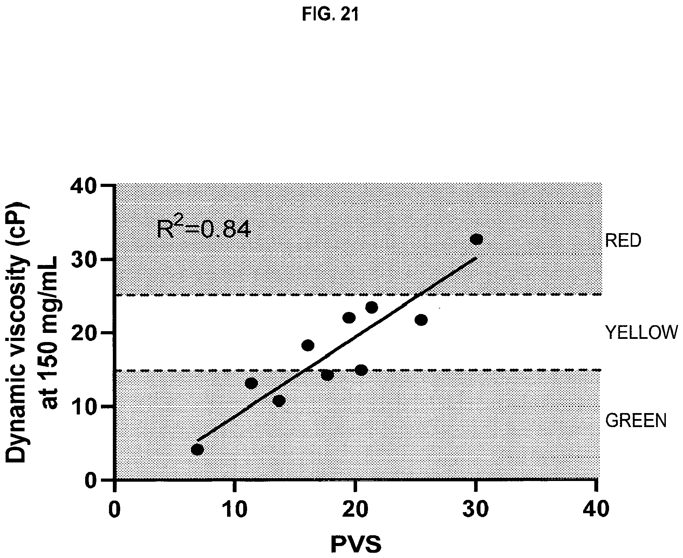

Model validation using the test set (data blinded correlation) for dynamic viscosity, is shown in **FIG. 21.** PVS (Eq. 5) and PAS (Eq. 6) models were validated using data from 10 mAbs (mix of IgG1 and IgG4). Data was blinded from the user to ensure unbiased validation. Color coding for risk ranking (divided by dashed lines and labeled as to color) is based on historical developmental goals throughout the biopharma industry and do not reflect any regulatory requirements.

| mAb ID | PVS | Dynamic viscosity (cP) at 150 mg/mL |
|--------|-----|-------------------------------------|
| mAb A | 13.7 | 10.7 |
| mAb B | 20.5 | 14.9 |
| mAb C | 19.5 | 22 |
| mAb D | 6.9 | 4.1 |
| mAb E | 11.4 | 13.1 |
| mAb F | 17.7 | 14.2 |
| mAb G | 25.5 | 21.7 |
| mAb H | 30.1 | 32.6 |
| mAb I | 21.4 | 23.4 |
| mAb J | 16.1 | 18.2 |

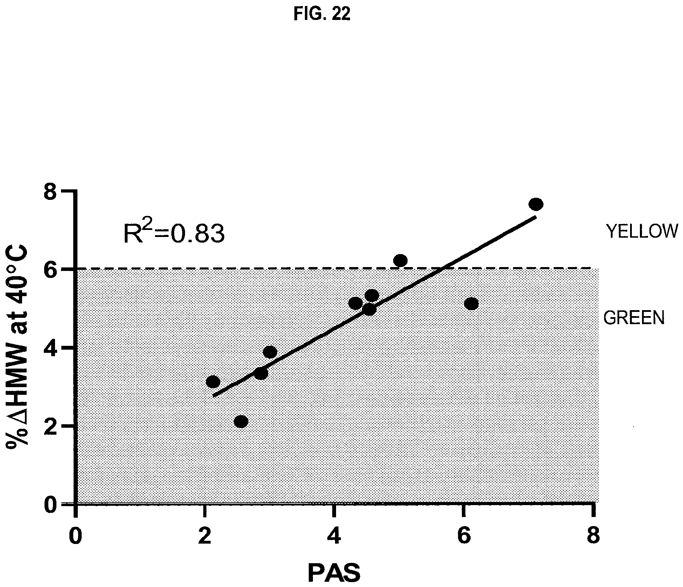

Model validation using the test set (data blinded correlation) for high-molecular-weight (HMW) species formation is shown in **FIG. 22.** %ΔHMW was calculated as:

$$\%\Delta\text{HMW} = C_0 + C_1 \times Z_{VL}^* + C_2 \times Z_{CH1}^* + C_3 \times \text{RMSD} + C_4 \times \text{HI} + C_5 \times D_{Fab} + C_6 \times \text{PI}_{Sequence}. \text{PVS}$$

and PAS models were validated using data from 10 mAbs (mix of IgG1 and IgG4). Data was blinded from the user to ensure unbiased validation.

Color coding for risk ranking (divided by dashed lines and labeled as to color) is based on historical developmental goals throughout biopharma industry and do not reflect any regulatory requirements.

| mAb ID | PAS | %ΔHMW at 40°C |
|--------|------|----------------|
| mAb A | 6.12 | 5.11 |
| mAb B | 4.55 | 4.97 |
| mAb C | 7.12 | 7.65 |
| mAb D | 4.34 | 5.12 |
| mAb E | 3.01 | 3.88 |
| mAb F | 2.56 | 2.11 |
| mAb G | 2.13 | 3.12 |
| mAb H | 2.87 | 3.34 |
| mAb I | 4.59 | 5.32 |
| mAb J | 5.03 | 6.22 |

**2. Results**

Predicted scores for both aggregation and viscosity were highly correlated (R2 values of above 0.8) to the validation experimental data. Strong statistical correlations further improve confidence in both prediction models and underlying AI algorithms.

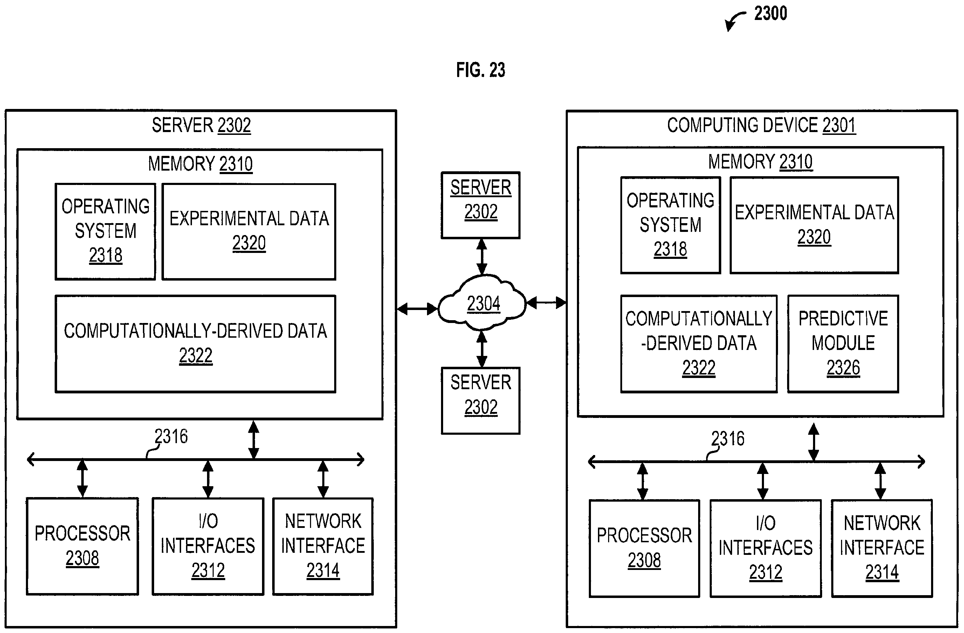

**FIG. 23** is a block diagram depicting an environment **2300** comprising nonlimiting examples of a computing device **2301** and a server **2302** connected through a network **2304.** In an aspect, some or all steps of any described method may be performed on a computing device as described herein. The computing device **2301** can comprise one or multiple computers configured to store one or more of experimental data **2320,** computationally-derived data **2322,** a predictive module **2326** (e.g., the ML module **230,** including any ancillary training modules), and the like. The server **2302** can comprise one or multiple computers configured to store the experimental data **2320** and/or the computationally-derived data **2322.** Multiple servers **2302** can communicate with the computing device **2301** via the through the network **2304.** In an embodiment, the server **2302** may comprise a repository for data generated by one or more experiments.

The computing device **2301** and the server **2302** can be a digital computer that, in terms of hardware architecture, generally includes a processor **2308,** memory system **2310,** input/output (I/O) interfaces **2312,** and network interfaces **2314.** These components (**2308, 2310, 2312**, and **2314**) are communicatively coupled via a local interface **2316.** The local interface **2316** can be, for example, but not limited to, one or more buses or other wired or wireless connections, as is known in the art. The local interface **2316** can have additional elements, which are omitted for simplicity, such as controllers, buffers (caches), drivers, repeaters, and receivers, to enable communications. Further, the local interface may include address, control, and/or data connections to enable appropriate communications among the aforementioned components.

The processor **2308** can be a hardware device for executing software, particularly that stored in memory system **2310.** The processor **2308** can be any custom made or commercially available processor, a central processing unit (CPU), an auxiliary processor among several processors associated with the computing device **2301** and the server **2302,** a semiconductor-based microprocessor (in the form of a microchip or chip set), or generally any device for executing software instructions. When the computing device **2301** and/or the server **2302** is in operation, the processor **2308** can be configured to execute software stored within the memory system **2310,** to communicate data to and from the memory system **2310,** and to generally control operations of the computing device **2301** and the server **2302** pursuant to the software.

The I/O interfaces **2312** can be used to receive user input from, and/or for providing system output to, one or more devices or components. User input can be provided via, for example, a keyboard and/or a mouse. System output can be provided via a display device and a printer (not shown). I/O interfaces **2312** can include, for example, a serial port, a parallel port, a Small Computer System Interface (SCSI), an infrared (IR) interface, a radio frequency (RF) interface, and/or a universal serial bus (USB) interface.

The network interface **2314** can be used to transmit and receive from the computing device **2301** and/or the server **2302** on the network **2304.** The network interface **2314** may include, for example, a 10BaseT Ethernet Adaptor, a 100BaseT Ethernet Adaptor, a LAN PHY Ethernet Adaptor, a Token Ring Adaptor, a wireless network adapter (e.g., WiFi, cellular, satellite), or any other suitable network interface device. The network interface **2314** may include address, control, and/or data connections to enable appropriate communications on the network **2304.**

The memory system **2310** can include any one or combination of volatile memory elements (e.g., random access memory (RAM, such as DRAM, SRAM, SDRAM, etc.)) and nonvolatile memory elements (e.g., ROM, hard drive, tape, CDROM, DVDROM, etc.). Moreover, the memory system **2310** may incorporate electronic, magnetic, optical, and/or other types of storage media. Note that the memory system **2310** can have a distributed architecture, where various components are situated remote from one another, but can be accessed by the processor **2308.**

The software in memory system **2310** may include one or more software programs, each of which comprises an ordered listing of executable instructions for implementing logical functions. In the example of **FIG. 23,** the software in the memory system **2310** of the computing device **2301** can comprise the experimental data **2320,** the computationally-derived data **2322,** the predictive module **2326,** and a suitable operating system (O/S) **2318.** In the example of **FIG. 23,** the software in the memory system **2310** of the server **2302** can comprise, the experimental data **2320,** the computationally-derived data **2322,** and a suitable operating system (O/S) **2318.** The operating system **2318** essentially controls the execution of other computer programs and provides scheduling, input-output control, file and data management, memory management, and communication control and related services.

For purposes of illustration, application programs and other executable program components such as the operating system **2318** are illustrated herein as discrete blocks, although it is recognized that such programs and components can reside at various times in different storage components of the computing device **2301** and/or the server **2302.** An implementation of the predictive module **2326** can be stored on or transmitted across some form of computer readable media. Any of the disclosed methods can be performed by computer readable instructions embodied on computer readable media. Computer readable media can be any available media that can be accessed by a computer. By way of example and not meant to be limiting, computer readable media can comprise "computer storage media" and "communications media." "Computer storage media" can comprise volatile and nonvolatile, removable and non-removable media implemented in any methods or technology for storage of information such as computer readable instructions, data structures, program modules, or other data. Exemplary computer storage media can comprise RAM, ROM, EEPROM, flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by a computer.

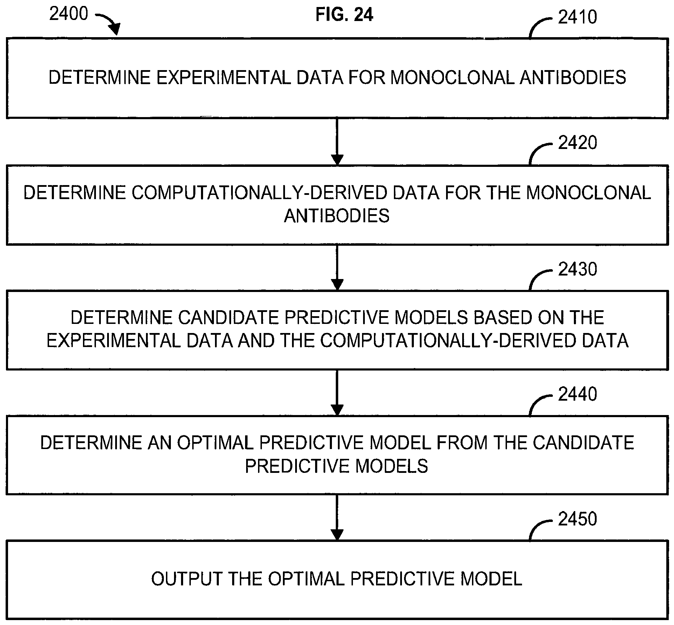

In an embodiment, the predictive module **2326** may be configured to perform a method **2400,** shown in **FIG. 24.** The method **2400** may be performed in whole or in part by a single computing device, a plurality of electronic devices, and the like. The method **2400** may comprise, at **2410,** determining experimental data associated with one or more monoclonal antibodies (mAbs). The the one or more mAbs may include one or more of an IgG1 antibody or an IgG4 antibody. The experimental data may include experimental viscosity data. The experimental viscosity data may include one or more of dynamic viscosity values or kinematic viscosity values.

Determining the experimental data associated with the one or more mAbs may include: measuring, based on a solution of each of the one or more mAbs and a viscometer, at least one of a dynamic viscosity value or a kinematic viscosity value.

The experimental data may include experimental aggregation data. The experimental aggregation data may include high-molecular-weight (HMW) species formation data for each mAb of the one or more mAbs. Determining the experimental data associated with the one or more mAbs may include: measuring, based on a solution of each of the one or more mAbs and size-exclusion chromatography (SEC), an amount of HMW species formation over time.

At **2420,** determining computationally-derived data associated with the one or more mAbs, wherein the computationally-derived data comprises one or more computational parameters weighted based on accessible surfaces (ASAs) of one or more residues of the one or more mAbs. The computationally-derived data may include charge data associated with one or more regions associated with a sequence of the one or more mAbs, modified charge data associated with the one or more regions based on a solvent accessible surface of a residue in a homology model of the one or more mAbs, a hydrophobicity index (HI), a dipole moment, or an isoelectric point (pI). Determining the computationally-derived data associated with the one or more mAbs may include full-antibody homology modeling of a sequence of the one or more mAbs or antigen-binding fragment (Fab) region modeling of the Fab sequence of the one or more mAbs.

Determining the computationally-derived data associated with the one or more mAbs may include: determining, based on a homology model of the one or more mAbs, one or more charge values associated with one or more residues in one or more regions of the one or more mAbs, determining, based on the homology model of the one or more mAbs, a solvent accessible surface (SAS) of the one or more residues in the one or more regions, adjusting, based on a weighting factor calculated using the SAS of the one or more residues relative to a total SAS associated with the one or more mAbs, the one or more charge values associated with the one or more residues, and determining, based on the homology model of the one or more mAbs and the adjusted one or more charge values associated with the one or more residues, a charge value associated with each region of the one or more regions.

The computationally-derived data may include charge data associated with one or more regions associated with a sequence of the one or more mAbs, modified charge data associated with the one or more regions based on a solvent accessible surface of a residue in a homology model of the one or more mAbs, a hydrophobicity index (HI), a dipole moment, an isoelectric point (pI), an aggregation propensity (AP), or a descriptor of conformational stability. The descriptor of conformational stability may include a backbone root mean square deviation (RMSD) of a conformational structure relative to an initial structure after rigid-body alignment. Determining the computationally-derived data associated with the one or more mAbs may include one or more Molecular Dynamics (MD) simulations associated with the one or more mAbs.

At **2430,** determining, based on the experimental data and the computationally-derived data, a plurality of candidate predictive models. Determining, based on the experimental data and the computationally-derived data, the plurality of candidate predictive models may include: identifying one or more experimental parameters of the experimental data as dependent variables, identifying one or more computational parameters of the computationally-derived data as independent variables, and determining, based on a stepwise regression algorithm, based on the dependent variables, and based on the intendent variables, the plurality of candidate predictive models.

At **2440,** determining an optimal predictive model from the plurality of candidate predictive models. Determining the optimal predictive model from the plurality of candidate predictive models may include: determining, for each candidate predictive model of the plurality of candidate predictive models, an Akaike Information Criterion (AIC) score, and determining, as the optimal predictive model, the candidate predictive model of the plurality of candidate predictive models associated with the highest AIC score.

Determining the optimal predictive model from the plurality of candidate predictive models may include: determining, as the optimal predictive model, the candidate predictive model of the plurality of candidate predictive models associated with a lowest error in predicting a viscosity score of a mAb excluded from the experimental data and the computationally-derived data.

Determining the optimal predictive model from the plurality of candidate predictive models may include: determining, as the optimal predictive model, the candidate predictive model of the plurality of candidate predictive models associated with a lowest error in predicting an aggregation score of a mAb excluded from the experimental data and the computationally-derived data.

At **2450,** outputting the optimal predictive model.

The method **2400** may also include receiving computationally-derived data associated with a query mAb, providing, to the optimal predictive model, the computationally-derived data, and determining, based on the optimal predictive model, a viscosity score associated with the query mAb. The method **2400** may include adjusting, based on the viscosity score, an appropriate formulation composition or protein engineering strategy to mitigate specific challenges with the drug candidate in development, for example, adjusting an amount of viscosity reducer of a solution associated with the query mAb.

The method **2400** may also include receiving computationally-derived data associated with a query mAb, providing, to the optimal predictive model, the computationally-derived data, and determining, based on the optimal predictive model, an aggregation score.

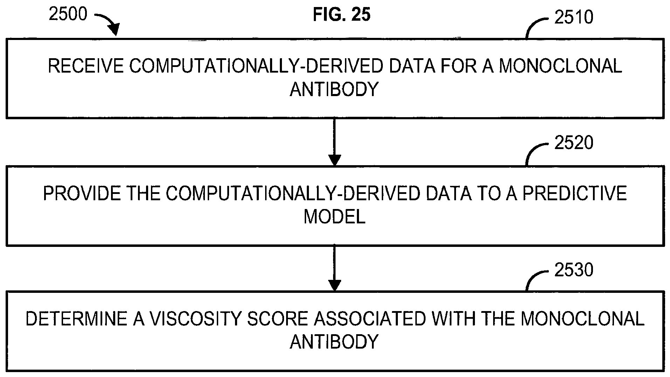

In an embodiment, the predictive module **2326** may be configured to perform a method **2500,** shown in **FIG. 25.** The method **2500** may be performed in whole or in part by a single computing device, a plurality of electronic devices, and the like. The method **2500** may comprise, at **2510,** receiving computationally-derived data associated with a monoclonal antibody (mAb). The computationally-derived data may include computationally-derived viscosity data. The computationally-derived viscosity data may include one or more of dynamic viscosity values or kinematic viscosity values.

At **2520,** providing, to a predictive model, the computationally-derived data.

At **2530** determining, based on the predictive model, a viscosity score associated with the mAb.

The method **2500** may also include adjusting, based on the viscosity score, an appropriate formulation composition or protein engineering strategy to mitigate specific challenges with the drug candidate in development, for example, adjusting an amount of viscosity reducer of a solution associated with the query mAb.

The method **2500** may also include receiving sequence data associated with the mAb, and determining, based on the sequence data, the computationally-derived data.

The method **2500** may also include: receiving computationally-derived data associated with a query mAb, providing, to the optimal predictive model, the computationally-derived data, and determining, based on the optimal predictive model, a viscosity score associated with the query mAb.

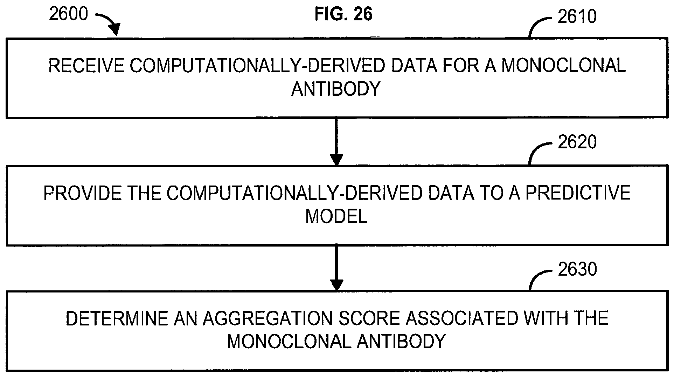

In an embodiment, the predictive module **2326** may be configured to perform a method **2600,** shown in **FIG. 26.** The method **2600** may be performed in whole or in part by a single computing device, a plurality of electronic devices, and the like. The method **2600** may comprise, at **2610,** receiving computationally-derived data associated with a monoclonal antibody (mAb). The computationally-derived data may include computationally-derived aggregation data. The computationally-derived aggregation data may include high-molecular-weight (HMW) species formation data for the mAb. The computationally-derived data may include charge data associated with one or more regions associated with a sequence of the mAb, modified charge data associated with the one or more regions based on a solvent accessible surface of a residue in a homology model of the mAb, a hydrophobicity index (HI), a dipole moment, an isoelectric point (pI), an aggregation propensity (AP), or a descriptor of conformational stability.

At **2620,** providing, to a predictive model, the computationally-derived data.

At **2630,** determining, based on the predictive model, an aggregation score associated with the mAb.

The method **2600** may also include receiving sequence data associated with the mAb, and determining, based on the sequence data, the computationally-derived data.

The method **2600** may also include determining an optimal predictive model from a plurality of candidate predictive models associated with a lowest error in predicting the aggregation score associated with the mAb.

The method **2600** may also include: receiving computationally-derived data associated with a query mAb, providing, to the optimal predictive model, the computationally-derived data associated with the query mAb, and determining, based on the optimal predictive model, an aggregation score associated with the query mAb

In view of the described apparatuses, systems, and methods and variations thereof, herein below are described certain more particularly described embodiments of the invention. These particularly recited embodiments should not however be interpreted to have any limiting effect on any different claims containing different or more general teachings described herein, or that the "particular" embodiments are somehow limited in some way other than the inherent meanings of the language literally used therein.

Embodiment 1: A method comprising: determining experimental data associated with one or more monoclonal antibodies (mAbs); determining computationally-derived data associated with the one or more mAbs, wherein the computationally-derived data comprises one or more computational parameters weighted based on accessible surfaces (ASAs) of one or more residues of the one or more mAbs; determining, based on the experimental data and the computationally-derived data, a plurality of candidate predictive models; determining an optimal predictive model from the plurality of candidate predictive models; and outputting the optimal predictive model.

Embodiment 2: The embodiment as in the embodiment 1, wherein wherein the one or more mAbs comprise one or more of an IgG1 antibody or an IgG4 antibody.

Embodiment 3: The embodiment of any of embodiments 1-2, wherein the experimental data comprises experimental viscosity data.

Embodiment 4: The embodiment of any of embodiments 1-3, wherein the experimental viscosity data comprises one or more of dynamic viscosity values or kinematic viscosity values.

Embodiment 5: The embodiment of any of embodiments 1-4, wherein determining the experimental data associated with the one or more mAbs comprises: measuring, based on a solution of each of the one or more mAbs and a viscometer, at least one of a dynamic viscosity value or a kinematic viscosity value.

Embodiment 6: The embodiment of any of embodiments 1-5, wherein the computationally-derived data comprises charge data associated with one or more regions associated with a sequence of the one or more mAbs, modified charge data associated with the one or more regions based on a solvent accessible surface of a residue in a homology model of the one or more mAbs, a hydrophobicity index (HI), a dipole moment, or an isoelectric point (pI).

Embodiment 7: The embodiment of any of embodiments 1-6, wherein determining the computationally-derived data associated with the one or more mAbs comprises full-antibody homology modeling of a sequence of the one or more mAbs or antigen-binding fragment (Fab) region modeling of the Fab sequence of the one or more mAbs.

Embodiment 8: The embodiment of any of embodiments 1-7, wherein determining the computationally-derived data associated with the one or more mAbs comprises: determining, based on a homology model of the one or more mAbs, one or more charge values associated with one or more residues in one or more regions of the one or more mAbs; determining, based on the homology model of the one or more mAbs, a solvent accessible surface (SAS) of the one or more residues in the one or more regions; adjusting, based on a weighting factor calculated using the SAS of the one or more residues relative to a total SAS associated with the one or more mAbs, the one or more charge values associated with the one or more residues; and determining, based on the homology model of the one or more mAbs and the adjusted one or more charge values associated with the one or more residues, a charge value associated with each region of the one or more regions.

Embodiment 9: The embodiment of any of embodiments 1-8, wherein determining, based on the experimental data and the computationally-derived data, the plurality of candidate predictive models comprises: identifying one or more experimental parameters of the experimental data as dependent variables; identifying one or more computational parameters of the computationally-derived data as independent variables; and determining, based on a stepwise regression algorithm, based on the dependent variables, and based on the intendent variables, the plurality of candidate predictive models.

Embodiment 10: The embodiment of any of embodiments 1-9, wherein determining the optimal predictive model from the plurality of candidate predictive models comprises: determining, for each candidate predictive model of the plurality of candidate predictive models, an Akaike Information Criterion (AIC) score; and determining, as the optimal predictive model, the candidate predictive model of the plurality of candidate predictive models associated with the highest AIC score.

Embodiment 11: The embodiment of any of embodiments 1-10, wherein determining the optimal predictive model from the plurality of candidate predictive models comprises: determining, as the optimal predictive model, the candidate predictive model of the plurality of candidate predictive models associated with a lowest error in predicting a viscosity score of a mAb excluded from the experimental data and the computationally-derived data.

Embodiment 12: The embodiment of any of embodiments 1-11, further comprising: receiving computationally-derived data associated with a query mAb; providing, to the optimal predictive model, the computationally-derived data; and determining, based on the optimal predictive model, a viscosity score associated with the query mAb.

Embodiment 13: The embodiment as in the embodiment 12, further comprising: adjusting, based on the viscosity score, an appropriate formulation composition or protein engineering strategy to mitigate specific challenges with the drug candidate in development, for example, adjusting an amount of viscosity reducer of a solution associated with the query mAb.

Embodiment 14: The embodiment of any of embodiments 1-13, wherein the experimental data comprises experimental aggregation data.

Embodiment 15: The embodiment as in the embodiment 14, wherein the experimental aggregation data comprises high-molecular-weight (HMW) species formation data for each mAb of the one or more mAbs.

Embodiment 16: The embodiment of any of embodiments 1-15, wherein determining the experimental data associated with the one or more mAbs comprises: measuring, based on a solution of each of the one or more mAbs and size-exclusion chromatography (SEC), an amount of HMW species formation over time.

Embodiment 17: The embodiment of any of embodiments 1-16, wherein determining the experimental data associated with the one or more mAbs comprises: measuring, based on a solution of each of the one or more mAbs and size-exclusion chromatography (SEC), an amount of HMW species formation over time.

Embodiment 18: The embodiment of any of embodiments 1-17, wherein the descriptor of conformational stability comprises a backbone root mean square deviation (RMSD) of a conformational structure relative to an initial structure after rigid-body alignment.

Embodiment 19: The embodiment of any of embodiments 1-18, wherein determining the computationally-derived data associated with the one or more mAbs comprises one or more Molecular Dynamics (MD) simulations associated with the one or more mAbs.

Embodiment 20: The embodiment of any of embodiments 1-19, wherein determining the optimal predictive model from the plurality of candidate predictive models comprises: determining, as the optimal predictive model, the candidate predictive model of the plurality of candidate predictive models associated with a lowest error in predicting an aggregation score of a mAb excluded from the experimental data and the computationally-derived data.

Embodiment 21: The embodiment of any of embodiments 1-20, further comprising: receiving computationally-derived data associated with a query mAb; providing, to the optimal predictive model, the computationally-derived data; and determining, based on the optimal predictive model, an aggregation score.

Embodiment 22: The embodiment of any of embodiments 1-21, further comprising adjusting, based on the aggregation score, an appropriate formulation composition or protein engineering strategy to mitigate specific challenges with the drug candidate in development, for example, adjusting an amount of aggregation reducer of a solution associated with the query mAb.

Embodiment 23: A method comprising: receiving computationally-derived data associated with a monoclonal antibody (mAb); providing, to a predictive model, the computationally-derived data; and determining, based on the predictive model, a viscosity score associated with the mAb.

Embodiment 24: The embodiment as in the embodiment 23, further comprising: adjusting, based on the viscosity score, an appropriate formulation composition or protein engineering strategy to mitigate specific challenges with the drug candidate in development, for example, adjusting an amount of viscosity reducer of a solution associated with the query mAb.

Embodiment 25: The embodiment of any of embodiments 23-24, further comprising: receiving sequence data associated with the mAb; and determining, based on the sequence data, the computationally-derived data.

Embodiment 26: The embodiment of any of embodiments 23-25, wherein the computationally-derived data comprises computationally-derived viscosity data.

Embodiment 27: The embodiment of any of embodiments 23-26, wherein the computationally-derived data comprises computationally-derived viscosity data.

Embodiment 28: The embodiment of any of embodiments 23-27, further comprising: receiving computationally-derived data associated with a query mAb; providing, to the optimal predictive model, the computationally-derived data; and determining, based on the optimal predictive model, a viscosity score associated with the query mAb.

Embodiment 29: A method comprising: receiving computationally-derived data associated with a monoclonal antibody (mAb); and providing, to a predictive model, the computationally-derived data; and determining, based on the predictive model, an aggregation score associated with the mAb.

Embodiment 30: The embodiment as in the embodiment 29, further comprising: adjusting, based on the aggregation score, an appropriate formulation composition or protein engineering strategy to mitigate specific challenges with the drug candidate in development, for example, adjusting an amount of aggregation reducer of a solution associated with the query mAb.

Embodiment 31: The embodiment of any of embodiments 29-30, further comprising: receiving sequence data associated with the mAb; and determining, based on the sequence data, the computationally-derived data.

Embodiment 32: The embodiment of any of embodiments 29-31, wherein the computationally-derived data comprises computationally-derived aggregation data.

Embodiment 33: The embodiment of any of embodiments 29-32, wherein the computationally-derived aggregation data comprises high-molecular-weight (HMW) species formation data for the mAb.

Embodiment 34: The embodiment of any of embodiments 29-33, wherein the computationally-derived data comprises charge data associated with one or more regions associated with a sequence of the mAb, modified charge data associated with the one or more regions based on a solvent accessible surface of a residue in a homology model of the mAb, a hydrophobicity index (HI), a dipole moment, an isoelectric point (pI), an aggregation propensity (AP), or a descriptor of conformational stability.

Embodiment 35: The embodiment of any of embodiments 29-34, further comprising determining an optimal predictive model from a plurality of candidate predictive models associated with a lowest error in predicting the aggregation score associated with the mAb.

Embodiment 36: The embodiment of any of embodiments 29-35, further comprising: receiving computationally-derived data associated with a query mAb; providing, to the optimal predictive model, the computationally-derived data associated with the query mAb; and determining, based on the optimal predictive model, an aggregation score associated with the query mAb.

While the methods and systems have been described in connection with preferred embodiments and specific examples, it is not intended that the scope be limited to the particular embodiments set forth, as the embodiments herein are intended in all respects to be illustrative rather than restrictive.

Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is in no way intended that an order be inferred, in any respect. This holds for any possible non-express basis for interpretation, including: matters of logic with respect to arrangement of steps or operational flow; plain meaning derived from grammatical organization or punctuation; the number or type of embodiments described in the specification.

Those skilled in the art will recognize, or be able to ascertain using no more than routine experimentation, many equivalents to the specific embodiments of the method and compositions described herein. Such equivalents are intended to be encompassed by the following claims.

**Claims**

1. A computer-implemented method of generating predictive model for predicting physical properties of one or more monoclonal antibodies (mAbs) comprising:

   determining experimental data associated with one or more monoclonal antibodies (mAbs);
   determining computationally-derived data associated with the one or more mAbs, wherein the computationally-derived data comprises one or more computational parameters, wherein the one or more computational parameters comprises one or more charge values associated with one or more residues in one or more regions of the one or more mAbs;
   calculating, based on a solvent accessible surface (SAS) of the one or more residues in the one or more regions of the one or more mAbs, a weighting factor;
   adjusting, based on the weighting factor the one or more charge values associated with the one or more residues;
   determining, based on the adjusted one or more charge values associated with the one or more residues, a charge value associated with each region of the one or more regions; and
   generating, based on the experimental data and the computationally-derived data, a predictive model, wherein the computationally-derived data comprises the charge values associated with each region of the one or more regions.

2. The computer-implemented method of claim 1, wherein the one or more mAbs comprise one or more of an IgG1 antibody or an IgG4 antibody.

3. The computer-implemented method of claim 1, wherein the experimental data comprises experimental viscosity data and wherein the experimental viscosity data comprises one or more of dynamic viscosity values or kinematic viscosity values.

4. The computer-implemented method of claim 3, wherein determining the experimental data associated with the one or more mAbs comprises measuring, based on a solution of each of the one or more mAbs and a viscometer, at least one of a dynamic viscosity value or a kinematic viscosity value.

5. The computer-implemented method of any one of the preceding claims, wherein the one or more regions are associated with a sequence of the one or more mAbs.

6. The computer-implemented method of any one of the preceding claims, wherein determining the computationally-derived data associated with the one or more mAbs comprises full-antibody homology modeling of a sequence of the one or more mAbs or antigen-binding fragment (Fab) region modeling of the Fab sequence of the one or more mAbs.

7. The computer-implemented method of any one of the preceding claims, wherein calculating, based on a solvent accessible surface (SAS) of the one or more residues in the one or more regions of the one or more mAbs, the weighting factor comprises:

determining, based on a homology model of the one or more mAbs, the one or more charge values associated with the one or more residues in one or more regions of the one or more mAbs; and
determining, based on the homology model of the one or more mAbs, the solvent accessible surface (SAS) of the one or more residues in the one or more regions.

8. The computer-implemented method of any one of the preceding claims, wherein generating, based on the experimental data and the computationally-derived data, the predictive model comprises:

identifying one or more experimental parameters of the experimental data as dependent variables;
identifying one or more computational parameters of the computationally-derived data as independent variables; and
generating, based on a stepwise regression algorithm, based on the dependent variables, and based on the intendent variables, the predictive model.

9. The computer-implemented method of any one of the preceding claims further comprises:
determining, for the predictive model an Akaike Information Criterion (AIC) score.

10. The computer-implemented method of any one of the preceding claims, further comprising:

receiving computationally-derived data associated with a query mAb;
providing, to the predictive model, the computationally-derived data; and
determining, based on the predictive model, a viscosity score associated with the query mAb.

11. The computer-implemented method of claim 9, further comprising:
adjusting, based on the viscosity score, a formulation composition associated with the query mAb.

12. The computer-implemented method of any one of the preceding claims, wherein determining the computationally-derived data associated with the one or more mAbs comprises one or more Molecular Dynamics (MD) simulations associated with the one or more mAbs.

13. The computer-implemented method of any one of the preceding claims, wherein generating, based on the experimental data and the computationally-derived data, the predictive model comprises:

generating a training data set comprising at least a portion of the experimental data and at least a portion of the computationally-derived data, wherein the at least the portion of the experimental data comprises direct measurements of antibody solution viscosity and the at least the portion of the computationally-derived data comprises one or more charge values of residues on antibodies of the antibody solution determined via computational modeling;

extracting, based on the training data set, a plurality of parameters; and

training, based on the training data set and the plurality of parameters, a machine learning-based classification model configured to predict a viscosity of an antibody.

14. The computer-implemented method of claim 13, wherein the plurality of parameters comprises one or more of: a SAS adjusted charge on a $V_L$ region, a SAS adjusted charge on a $V_H$ region, a SAS adjusted charge on a $C_L$ region, a SAS adjusted charge on a $C_H1$ region, a SAS adjusted charge on a hinge region, a SAS adjusted charge on a $C_H2$ region, a SAS adjusted charge on a $C_H3$ region, or a hydrophobicity index.

15. The computer-implemented method of claim 13, wherein the machine learning-based classification model configured to predict a viscosity of an antibody is configured to predict a likelihood that one or more computational parameters of a novel antibody are associated with a low or a high viscosity score.

**FIG. 1**

```
┌─────────────────────────────┐          ┌──────────────┐          ┌─────────────────────────────┐
│  TRAINING DATA SET 210A     │          │              │          │        ML MODULE            │
│                             │          │              │          │          230                │
│  ┌───────────────────────┐  │          │              │          │  ┌───────────────────────┐  │
│  │    EXPERIMENTAL       │  │          │              │          │  │                       │  │
│  │    PARAMETERS         │  │          │              │          │  │ MACHINE LEARNING-BASED│  │
│  └───────────────────────┘  │   ⇨      │              │          │  │ CLASSIFICATION MODEL  │  │
│                             │          │              │          │  │         240A          │  │
│  ┌───────────────────────┐  │          │              │          │  │                       │  │
│  │   COMPUTATIONAL       │  │          │              │          │  └───────────────────────┘  │
│  │   PARAMETERS          │  │          │  TRAINING    │          │                             │
│  └───────────────────────┘  │          │  MODULE      │   ⇨      │           O                 │
└─────────────────────────────┘          │   220        │          │           O                 │
              O                          │              │          │           O                 │
              O                          │              │          │                             │
              O                          │              │          │  ┌───────────────────────┐  │
┌─────────────────────────────┐          │              │          │  │                       │  │
│  TRAINING DATA SET 210N     │          │              │          │  │ MACHINE LEARNING-BASED│  │
│                             │          │              │          │  │ CLASSIFICATION MODEL  │  │
│  ┌───────────────────────┐  │          │              │          │  │         240N          │  │
│  │    EXPERIMENTAL       │  │   ⇨      │              │          │  │                       │  │
│  │    PARAMETERS         │  │          │              │          │  └───────────────────────┘  │
│  └───────────────────────┘  │          │              │          │                             │
│  ┌───────────────────────┐  │          │              │          │                             │
│  │   COMPUTATIONAL       │  │          │              │          │                             │
│  │   PARAMETERS          │  │          │              │          │                             │
│  └───────────────────────┘  │          │              │          │                             │
└─────────────────────────────┘          └──────────────┘          └─────────────────────────────┘
```

~200

**FIG. 2**

**FIG. 3**

```
┌─────────────────┐        ┌─────────────────┐        ┌─────────────────┐
│   UNCLASSIFIED  │        │                 │        │                 │
│  COMPUTATIONAL  │  ═══▷  │    ML MODULE    │  ═══▷  │ PREDICTION RESULT│
│   PARAMETERS    │        │       230       │        │       420       │
│       410       │        │                 │        │                 │
└─────────────────┘        └─────────────────┘        └─────────────────┘
```

FIG. 4

FIG. 5

EP 4 435 794 A2

| Antibody | Isotype | $Z_{VL}$ | $Z_{VH}$ | $Z_{CL}$ | $Z_{CH1}$ | $Z_{Hinge}$ | $Z_{CH2}$ | $Z_{CH3}$ | $Z_{mAb}$ | $Z'_{VL}$ | $Z'_{VH}$ | $Z'_{CL}$ | $Z'_{CH1}$ | $Z'_{Hinge}$ | $Z'_{CH2}$ | $Z'_{CH3}$ |
|---|---|---|---|---|---|---|---|---|---|---|---|---|---|---|---|---|
| mAb1 | IgG1 | 3.02 | 4.70 | -2.22 | 4.78 | -0.56 | 4.29 | -0.04 | 30.50 | 0.0155 | 0.0191 | -0.0029 | 0.0165 | -0.0011 | 0.0185 | 0.0051 |
| mAb2 | IgG1 | 3.09 | 3.78 | -2.18 | 5.82 | -0.41 | 4.22 | 0.01 | 30.18 | 0.0172 | 0.0174 | 0.0029 | 0.0101 | 0.0019 | 0.0219 | 0.0037 |
| mAb3 | IgG1 | 3.01 | 4.38 | -2.33 | 5.50 | -0.37 | 4.17 | 0.20 | 29.89 | 0.0154 | 0.0205 | 0.0024 | 0.0134 | -0.0007 | 0.0162 | 0.0021 |
| mAb4 | IgG1 | 3.25 | 3.15 | -2.27 | 5.42 | -0.43 | 4.51 | -0.17 | 28.41 | 0.0098 | 0.0232 | 0.0020 | 0.0179 | -0.0010 | 0.0176 | 0.0016 |
| mAb5 | IgG4 | 1.92 | 2.26 | -1.97 | 4.58 | 0.23 | 2.89 | -1.44 | 18.63 | 0.0116 | 0.0166 | 0.0021 | 0.0105 | 0.0015 | 0.0165 | -0.0114 |
| mAb6 | IgG1 | 4.11 | 3.31 | -2.20 | 5.22 | -0.64 | 4.62 | 0.06 | 30.83 | 0.0212 | 0.0158 | 0.0040 | 0.0138 | -0.0031 | 0.0172 | 0.0026 |
| mAb7 | IgG4 | 2.03 | 5.16 | -2.04 | 4.54 | 0.30 | 2.65 | -1.63 | 22.98 | 0.0127 | 0.0236 | -0.0001 | 0.0124 | 0.0013 | 0.0198 | -0.0071 |
| mAb8 | IgG4 | 3.03 | 3.45 | -2.12 | 4.74 | 0.23 | 2.69 | -1.38 | 21.53 | 0.0168 | 0.0200 | 0.0000 | 0.0085 | 0.0014 | 0.0211 | -0.0104 |
| mAb9 | IgG4 | 0.97 | 3.34 | -2.03 | 4.60 | -0.22 | 2.71 | -1.43 | 18.72 | 0.0014 | 0.0200 | -0.0007 | 0.0113 | 0.0014 | 0.0154 | -0.0088 |
| mAb10 | IgG4 | 3.06 | 3.57 | -2.07 | 3.98 | 0.13 | 2.36 | -1.67 | 21.08 | 0.0172 | 0.0102 | -0.0006 | 0.0089 | 0.0019 | 0.0153 | -0.0123 |
| mAb11 | IgG1 | 2.68 | 2.55 | -2.33 | 5.19 | -0.22 | 4.49 | -0.02 | 26.01 | 0.0153 | 0.0193 | 0.0022 | 0.0114 | 0.0009 | 0.0159 | 0.0047 |
| mAb12 | IgG4 | 4.04 | 3.84 | -2.42 | 4.21 | 0.10 | 2.49 | -1.56 | 22.92 | 0.0225 | 0.0191 | -0.0025 | -0.0132 | 0.0011 | 0.0201 | -0.0119 |
| mAb13 | IgG4 | 5.16 | 2.37 | -2.33 | 4.04 | 0.13 | 2.53 | -1.18 | 24.50 | 0.0270 | 0.0160 | 0.0005 | 0.0113 | 0.0021 | 0.0199 | -0.0105 |
| mAb14 | IgG4 | 4.27 | -0.57 | -2.05 | 3.96 | 0.05 | 2.89 | -1.52 | 17.06 | 0.0215 | 0.0036 | 0.0039 | 0.0112 | 0.0015 | 0.0175 | -0.0097 |
| mAb15 | IgG4 | 3.01 | 2.72 | -1.54 | 4.77 | 0.10 | 2.84 | -0.98 | 21.3 | 0.0142 | 0.0177 | -0.0008 | 0.0120 | 0.0014 | 0.0178 | -0.0110 |
| mAb16 | IgG4 | 4.04 | 3.55 | -2.01 | 4.07 | -0.50 | 2.46 | -0.67 | 23.14 | 0.0205 | 0.0176 | -0.0053 | 0.0097 | 0.0014 | 0.0168 | -0.0088 |

FIG. 6A

| Antibody | Isotype | HI | $D_{mAb}$ | $pI_{Sequence}$ | $pI_{Structure}$ | AP |
|---|---|---|---|---|---|---|
| mAb1 | IgG1 | 1.23 | 615.8 | 9.37 | 10.94 | -6275.85 |
| mAb2 | IgG1 | 1.22 | 622.095 | 9.29 | 9.62 | -6486.04 |
| mAb3 | IgG1 | 1.23 | 781.932 | 9.37 | 9.68 | -6402.95 |
| mAb4 | IgG1 | 1.06 | 358.01 | 9.11 | 9.44 | -6543.17 |
| mAb5 | IgG4 | 1.32 | 1046.12 | 7.76 | 8.29 | -5917.39 |
| mAb6 | IgG1 | 1.21 | 848.615 | 9.45 | 10.98 | -6677.57 |
| mAb7 | IgG4 | 1.35 | 2349.38 | 9.14 | 9.38 | -6153.84 |
| mAb8 | IgG4 | 1.13 | 1319.31 | 8.94 | 9.21 | -6607.31 |
| mAb9 | IgG4 | 1.11 | 1280.63 | 7.72 | 8.23 | -6437.43 |
| mAb10 | IgG4 | 1.28 | 1787.47 | 8.77 | 8.86 | -6369.26 |
| mAb11 | IgG1 | 1.10 | 271.935 | 9.14 | 9.47 | -7387.04 |
| mAb12 | IgG4 | 1.21 | 1866.28 | 8.77 | 9.06 | -6075.37 |
| mAb13 | IgG4 | 1.11 | 1623.68 | 9.10 | 9.46 | -6098.54 |
| mAb14 | IgG4 | 1.16 | 528.071 | 6.98 | 7.93 | -6242.53 |
| mAb15 | IgG4 | 1.14 | 1265.22 | 8.79 | 8.92 | -6674.37 |
| mAb16 | IgG4 | 1.09 | 1395.61 | 9.07 | 9.39 | -6258.63 |

FIG. 6B

| Antibody | Isotype | $Z_{VL}$ | $Z_{VH}$ | $Z_{CL}$ | $Z_{CH1}$ | $Z_{Fab}$ | $Z'_{VL}$ | $Z'_{VH}$ | $Z'_{CL}$ | $Z'_{CH1}$ | HI | $D_{mAb}$ | $pI_{sequence}$ | $pI_{structure}$ | AP | RMSD |
|---|---|---|---|---|---|---|---|---|---|---|---|---|---|---|---|---|
| mAb1 | IgG1 | 3.05 | 4.72 | -2.02 | 4.54 | 11.29 | 0.0267 | 0.0308 | -0.0063 | 0.0248 | 1.23 | 316.418 | 9.37 | 9.92 | -1963.86 | 1.872 |
| mAb2 | IgG1 | 3.14 | 3.72 | -2.16 | 4.67 | 10.36 | 0.0295 | 0.0311 | -0.0002 | 0.0240 | 1.22 | 250.287 | 9.29 | 9.95 | -1892.09 | 1.907 |
| mAb3 | IgG1 | 3.04 | 4.63 | -2.20 | 4.23 | 10.70 | 0.0279 | 0.0342 | 0.0047 | 0.0211 | 1.23 | 295.767 | 9.37 | 9.94 | -2084.41 | 2.294 |
| mAb4 | IgG1 | 3.22 | 3.47 | -1.95 | 4.37 | 10.11 | 0.0362 | 0.0518 | 0.0042 | 0.0231 | 1.06 | 372.093 | 9.11 | 9.84 | -2147.38 | 2.384 |
| mAb5 | IgG4 | 2.02 | 2.45 | -1.61 | 3.67 | 7.52 | 0.0189 | 0.0243 | 0.0038 | 0.0142 | 1.32 | 318.258 | 7.76 | 9.62 | -2132.05 | 1.785 |
| mAb6 | IgG1 | 4.08 | 3.60 | -2.39 | 4.46 | 10.64 | 0.0314 | 0.0268 | -0.0049 | 0.0215 | 1.21 | 460.961 | 9.45 | 10.06 | -2090.23 | 1.999 |
| mAb7 | IgG4 | 2.07 | 5.82 | -1.93 | 3.83 | 10.79 | 0.0154 | 0.0375 | -0.0047 | 0.0188 | 1.35 | 557.576 | 9.14 | 10.17 | -2043.86 | 1.970 |
| mAb8 | IgG4 | 3.15 | 3.15 | -1.55 | 3.69 | 9.43 | 0.0269 | 0.0279 | 0.0065 | 0.0184 | 1.13 | 467.066 | 8.94 | 10.01 | -2338.8 | 2.394 |
| mAb10 | IgG4 | 3.04 | 3.32 | -1.84 | 3.69 | 9.20 | 0.0235 | 0.0176 | 0.0053 | 0.0119 | 1.28 | 355.414 | 8.77 | 9.89 | -2276.99 | 2.201 |
| mAb11 | IgG1 | 2.19 | 2.72 | -1.90 | 4.48 | 8.49 | 0.0213 | 0.0396 | -0.0024 | 0.0210 | 1.10 | 347.14 | 9.14 | 9.74 | -2389.93 | 2.263 |
| mAb12 | IgG4 | 4.14 | 3.42 | -1.82 | 3.77 | 10.51 | 0.0354 | 0.0276 | -0.0021 | 0.0285 | 1.21 | 310.087 | 8.77 | 10.06 | -2092.8 | 2.334 |
| mAb13 | IgG4 | 5.26 | 2.30 | -2.07 | 3.43 | 9.91 | 0.0406 | 0.0237 | 0.0017 | 0.0213 | 1.11 | 314.203 | 9.10 | 10.13 | -2201.58 | 3.159 |
| mAb14 | IgG4 | 4.37 | -0.42 | -1.93 | 3.75 | 6.77 | 0.0346 | 0.0100 | 0.0011 | 0.0168 | 1.16 | 253.192 | 6.98 | 9.24 | -2254.63 | 2.674 |
| mAb15 | IgG4 | 3.07 | 2.92 | -2.01 | 3.77 | 8.74 | 0.0208 | 0.0266 | -0.0038 | 0.0232 | 1.14 | 300.056 | 8.79 | 9.81 | -2494.02 | 2.431 |

FIG. 7

FIG. 8

FIG. 9A

FIG. 9B

FIG. 9C

FIG. 10A

FIG. 10B

**FIG. 11A**

FIG. 11B

FIG. 11C

FIG. 12

FIG. 13

FIG. 14

FIG. 15

FIG. 16A

FIG. 16B

**FIG. 16C**

FIG. 17

FIG. 18A

FIG. 18B

FIG. 18C

**FIG. 19A**

FIG. 19B

FIG. 19C

FIG. 20

FIG. 21

FIG. 22

FIG. 23

EP 4 435 794 A2

**FIG. 24**

2400

2410

DETERMINE EXPERIMENTAL DATA FOR MONOCLONAL ANTIBODIES

2420

DETERMINE COMPUTATIONALLY-DERIVED DATA FOR THE MONOCLONAL ANTIBODIES

2430

DETERMINE CANDIDATE PREDICTIVE MODELS BASED ON THE EXPERIMENTAL DATA AND THE COMPUTATIONALLY-DERIVED DATA

2440

DETERMINE AN OPTIMAL PREDICTIVE MODEL FROM THE CANDIDATE PREDICTIVE MODELS

2450

OUTPUT THE OPTIMAL PREDICTIVE MODEL

**FIG. 25**

2500

2510

RECEIVE COMPUTATIONALLY-DERIVED DATA FOR A MONOCLONAL ANTIBODY

2520

PROVIDE THE COMPUTATIONALLY-DERIVED DATA TO A PREDICTIVE MODEL

2530

DETERMINE A VISCOSITY SCORE ASSOCIATED WITH THE MONOCLONAL ANTIBODY

2600

**FIG. 26**

2610

| RECEIVE COMPUTATIONALLY-DERIVED DATA FOR A MONOCLONAL ANTIBODY |

2620

| PROVIDE THE COMPUTATIONALLY-DERIVED DATA TO A PREDICTIVE MODEL |

2630

| DETERMINE AN AGGREGATION SCORE ASSOCIATED WITH THE MONOCLONAL ANTIBODY |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63108716

**Non-patent literature cited in the description**

- **COLIGAN et al.** Current Protocols in Immunology. John Wiley & Sons, 1991-1997
- **CRAMER et al.** *Quant. Struct-Act. Relat.,* 1998, vol. 7, 18-25